# EUROPEAN PATENT APPLICATION

(11) **EP 3 541 064 A1**
(43) Date of publication of application: **18.09.2019**
(21) Application number: 17869966.6
(22) Date of filing: 27.10.2017
(51) Int. Cl.: H04N 5/232, G03B 17/18

(54) **IMAGE PROCESSING DEVICE AND METHOD, AND PROGRAM**

(30) Priority: 10.11.2016 JP 2016219405
(71) Applicant: Sony Corporation, Tokyo 108-0075 (JP)
(72) Inventor: YOROZU, Shutaro, Tokyo 108-0075 (JP)
(74) Representative: D Young & Co LLP
(86) International application number: PCT/JP2017/038846
(87) International publication number: WO 2018/088236

(57) **Abstract**

The present technology relates to an image processing device and method, and a program that can improve usability.

The image processing device includes a setting unit that sets a flip function of inverting a display direction of an image to a predetermined direction to ON or OFF, and a control unit that inverts a screen associated with setting of the flip function in the predetermined direction and displays the inverted screen when an instruction to set the flip function to ON is received. The present technology can be applied to a digital camera.

## Description

### TECHNICAL FIELD

The present technology relates to an image processing device and method, and a program and, more particularly, to an image processing device and method, and a program that can improve usability.

### BACKGROUND ART

For example, a technology associated with a camera has been proposed in which an image of one view is laterally inverted and displayed when a half-width image in which two images having different views are cut out from an image acquired by unfolding a wide-view image and are arranged is displayed (for example, see Patent Document 1).

When imaging is performed using a camera to which such a technology is applied and which is installed in a vehicle and an acquired half-width image is displayed, scenes flow in the same direction in two areas having different views in the half-width image and thus the technology can be used for applications such as driving assist.

Further, when imaging is performed using a camera, an image may be captured with the camera gripped by a hand or with the camera fixed to a vehicle, a ceiling, or the like depending on applications for use. However, when a moving image is captured with the camera inverted, the top and bottom of an image or right and left channels of sound are inverted.

Therefore, a function of vertically inverting a recording image or exchanging right and left channels of sound on the assumption that an image is captured with a camera inverted has also been proposed.

### CITATION LIST

### PATENT DOCUMENT

Patent Document 1: Japanese Patent Application Laid-Open No. 2008-28778

### SUMMARY OF THE INVENTION

### PROBLEMS TO BE SOLVED BY THE INVENTION

However, in the above-mentioned technology, an imaging operation or the like in capturing an image with a camera inverted, for example, may be inconvenient and it cannot be said to improve usability by only vertically inverting a captured image.

The present technology is made in consideration of the above-mentioned circumstances and an objective thereof is to improve usability.

### SOLUTIONS TO PROBLEMS

According to an aspect of the present technology, there is provided an image processing device including: a setting unit that sets a flip function of inverting a display direction of an image to a predetermined direction to ON or OFF; and a control unit that inverts a screen associated with setting of the flip function in the predetermined direction and displays the inverted screen when an instruction to set the flip function to ON is received.

The control unit may invert the screen associated with setting of the flip function in a vertical direction and display the inverted screen when an instruction to set the flip function to ON is received.

The control unit may not display the screen associated with setting of the flip function when the flip function is set to OFF.

The screen associated with setting of the flip function may be an explanation screen for the flip function.

Explanation for behavior of an operation unit when the flip function is set to ON may be displayed on the explanation screen.

The screen associated with setting of the flip function maybe an ascertainment screen for setting of the flip function.

The setting unit may cancel setting of the flip function which has been performed before the ascertainment screen is displayed when cancellation of setting of the flip function is instructed in a state in which the ascertainment screen is displayed.

A cancellation button for cancelling setting of the flip function may be displayed on the ascertainment screen.

At least one of vertical inversion of an image acquired by imaging, exchange of right and left channels of collected sound acquired by sound collection, vertically inverted display of a display screen, or exchange of functions assigned to the operation units associated with different directions may be performed when the flip function is set to ON.

According to another aspect of the present technology, there is provided an image processing method or a program including steps of: setting a flip function of inverting a display direction of an image to a predetermined direction to ON or OFF; and inverting a screen associated with setting of the flip function in the predetermined direction and displaying the inverted screen when an instruction to set the flip function to ON is received.

According to the aspects of the present technology, a flip function of inverting a display direction of an image to a predetermined direction is set to ON or OFF and a screen associated with setting of the flip function is inverted in the predetermined direction and is displayed when an instruction to set the flip function to ON is received.

### EFFECTS OF THE INVENTION

According to the aspect of the present technology, it is possible to improve usability.

Incidentally, the present technology is not limited to the above-mentioned effects and may have any effect described in the present disclosure.

### BRIEF DESCRIPTION OF DRAWINGS

Fig. 1 is a diagram illustrating an example of an appearance configuration of an imaging device.
Fig. 2 is a diagram illustrating an example of an appearance configuration of the imaging device.
Fig. 3 is a diagram illustrating an example of an appearance configuration of the imaging device.
Fig. 4 is a diagram illustrating an example of an appearance configuration of the imaging device.
Fig. 5 is a diagram illustrating an example of a functional configuration of the imaging device.
Fig. 6 is a diagram describing flip setting.
Fig. 7 is a diagram describing flip setting.
Fig. 8 is a diagram describing flip setting.
Fig. 9 is a flowchart describing a flip setting process.
Fig. 10 is a diagram describing flip setting and capturing of a moving image.
Fig. 11 is a diagram describing flip setting and capturing of a moving image.
Fig. 12 is a flowchart describing an imaging process.
Fig. 13 is a diagram describing flip setting and outputting of an image.
Fig. 14 is a diagram describing flip setting and outputting of an image.
Fig. 15 is a flowchart describing an output process.
Fig. 16 is a diagram describing flip setting and behavior of a direction key.
Fig. 17 is a diagram describing flip setting and behavior of a direction key.
Fig. 18 is a diagram describing change of display associated with a DISP function.
Fig. 19 is a diagram describing change of display associated with a DISP function.
Fig. 20 is a diagram describing change of display associated with a DISP function.
Fig. 21 is a diagram illustrating a display example of a function setting screen.
Fig. 22 is a diagram illustrating a display example of a WB setting screen.
Fig. 23 is a diagram illustrating a display example of a menu screen.
Fig. 24 is a flowchart describing a key operating process.
Fig. 25 is a diagram illustrating an example of an appearance configuration of a remote operation unit.
Fig. 26 is a flowchart describing a command executing process.
Fig. 27 is a diagram illustrating an example of a configuration of a computer.
Fig. 28 is a diagram schematically illustrating an example of a configuration of an endoscopic surgery system.
Fig. 29 is a block diagram illustrating an example of a functional configuration of a camera head and a CCU which are illustrated in Fig. 28.

### MODE FOR CARRYING OUT THE INVENTION

Hereinafter, embodiments to which the present technology is applied will be described with reference to the accompanying drawings.

### <First Embodiment>

### <Example of Appearance Configuration of Imaging Device>

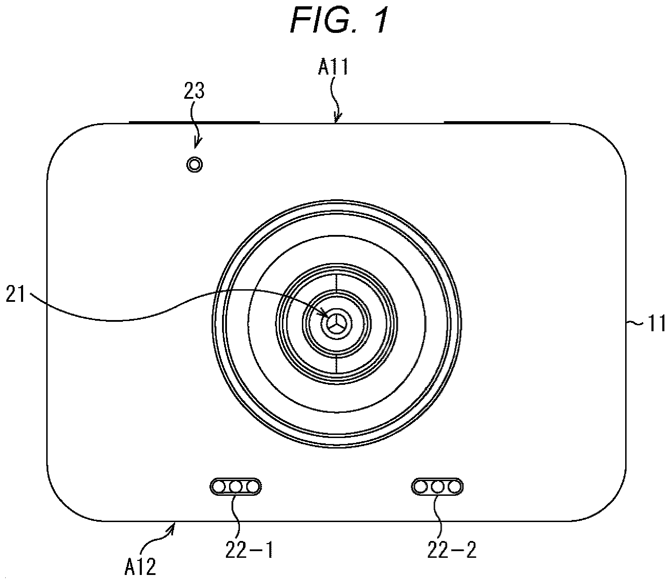

First, an example of an appearance configuration of an imaging device which is an example of an image processing device to which the present technology is applied will be described with reference to Figs. 1 to 4.

Fig. 1 illustrates a front view of the appearance of the imaging device.

An imaging device 11 includes, for example, a digital camera and the like, and is configured to perform imaging in a state in which a user who is a photographer takes the imaging device 11 with a hand and to perform imaging in a state in which the imaging device 11 is fixed to a bicycle, a ceiling, a wall, or the like.

A lens 21 that guides light from a subject to an imaging element which is not illustrated is provided on the front surface of the imaging device 11. Further, a sound collecting unit 22-1 and a sound collecting unit 22-2 which are constituted by microphones that collect ambient sound at the time of capturing a moving image are provided below the lens 21 on the front surface of the imaging device 11 in the drawing. Basically, sound collected by the sound collecting unit 22-1 becomes sound of a right channel and sound collected by the sound collecting unit 22-2 becomes sound of a left channel.

Incidentally, in the following description, the sound collecting unit 22-1 and the sound collecting unit 22-2 are also simply referred to as a sound collecting unit 22 when they are not distinguished from each other.

A tally lamp 23 is provided on the upper-left side of the front surface of the imaging device 11 in the drawing. The tally lamp 23 is turned on while the imaging device 11 is capturing a moving image and is turned off while the imaging device is not capturing a moving image.

Further, in generation, when a moving image or a still image is captured using the imaging device 11, imaging is performed in a state in which the top surface indicated by an arrow A11 faces the upper side in a vertical direction, that is, in a state in which the bottom surface indicated by an arrow A12 faces the ground surface.

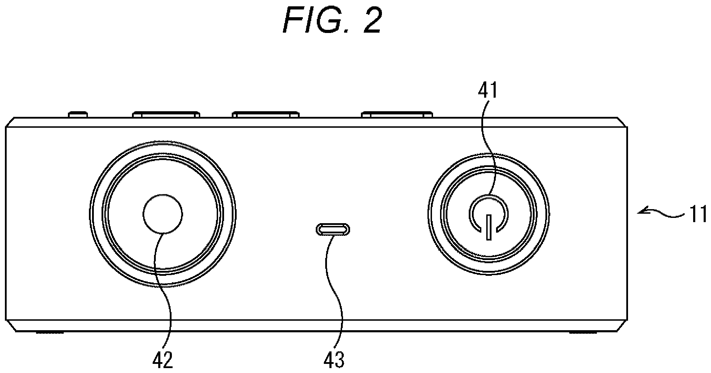

Accordingly, for example, as illustrated in Fig. 2, a power supply button 41, a shutter button 42, a sound output unit 43, and the like are provided on the top surface of the imaging device 11. Incidentally, Fig. 2 illustrates a top view of the imaging device 11 when seen from the upper side to the lower side in Fig. 1.

The power supply button 41 is a button which is operated when the imaging device 11 is powered on or off. The shutter button 42 is operated when a still image is captured, when capturing of a moving image is started, when imaging of a moving image is stopped, or the like. Further, the shutter button 42 functions as an Enter key (a selection button) for determining selection in a state in which a menu screen or the like is displayed on a display unit which is not illustrated.

The sound output unit 43 is constituted, for example, by a speaker and the like, and reproduces sound of a moving image at the time of reproduction of the moving image.

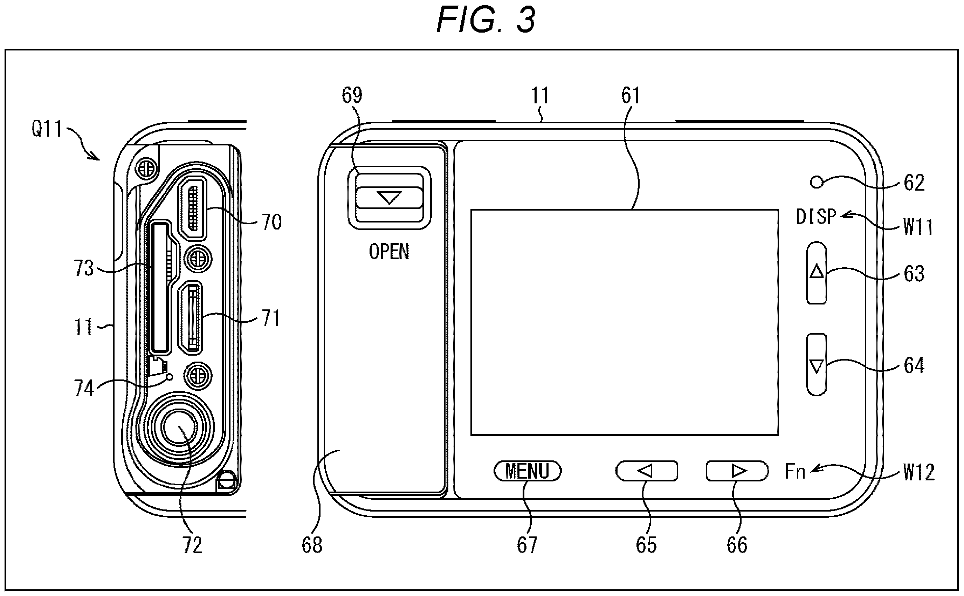

Further, on a surface opposite to the front surface of the imaging device 11 illustrated in Fig. 1, that is, a rear surface of the imaging device 11, for example, various buttons and the like are provided as illustrated in Fig. 3. Incidentally, in Fig. 3, the upper side of the imaging device 11 in Fig. 3 is the top surface side and the lower side of the imaging device 11 in Fig. 3 is the bottom surface side.

A display unit 61 which is constituted, for example, by a liquid crystal display (LCD) and the like, and which displays various images is provided on the rear surface of the imaging device 11.

Further, a tally lamp 62, an upward button 63, a downward button 64, a leftward button 65, a rightward button 66, and a menu button 67 are provided around the display unit 61.

Similarly to the tally lamp 23 which is provided on the front surface of the imaging device 11, the tally lamp 62 is turned on when a moving image is being captured and is turned off when a moving image is not being captured.

The upward button 63 is operated when an upward operation is performed on an image, a menu screen, or the like displayed on the display unit 61.

That is, the upward button 63 is operated, for example, when a cursor or the like on a menu screen is moved to the top end of the menu screen or when a scrolling operation for displaying an area close to the top end of an image acquired by imaging is performed, for example.

In the following description, it is particularly assumed that a direction directed from the center to the top end of an image, a menu screen, or the like is also referred to as a screen upward direction, and similarly, directions directed from the center to the bottom, the left end, and the right end of the image, the menu screen, or the like are also referred to as a screen downward direction, a screen leftward direction, and a screen rightward direction, respectively. For example, the screen upward direction is an upward direction with respect to an image, a menu screen, or the like, that is, on the image or the menu screen.

Further, in the following description, it is assumed that functions of performing operations associated with the screen upward direction, the screen downward direction, the screen leftward direction, and the screen rightward direction on an image, a menu screen, or the like are also referred to as an upward operating function, a downward operating function, a leftward operating function, and a rightward operating function, respectively.

Accordingly, the upward operating function such as an operation associated with the upward direction with respect to a display screen such as an image or a menu screen, that is, a scrolling operation of moving a cursor or the like in the screen upward direction or displaying an area close to an end in the screen upward direction, is assigned to the upward button 63.

Other than, the upward button 63 is also operated when display on the display unit 61 is switched at the time of capturing an image or reproducing an image. That is, when the upward button 63 is operated, the display on the display unit 61 is switched depending on the operation on the upward button 63 in a state in which marks or the like indicating various settings are displayed as little as possible, a state in which marks or the like indicating various settings are displayed more, a state in which a histogram associated with an image is displayed, and the like.

In the following description, a function of switching the display on the display unit 61 at the time of capturing an image or reproducing an image is referred to as a DISP function.

In this way, the upward operating function and the DISP function are assigned to the upward button 63.

Characters "DISP" indicating the DISP function are printed above the upward button 63 in the drawing, that is, in a part indicated by an arrow W11, on the rear surface of the imaging device 11. A user (a person) can instantaneously understand that the DISP function is assigned to the upward button 63 by seeing the printed characters.

The downward button 64 is disposed below the upward button 63 in the drawing, that is, on the bottom surface side thereof. The downward button 64 is operated when a screen downward operation is performed. That is, a downward operating function is assigned to the downward button 64.

Further, an arbitrary function among a plurality of predetermined functions in addition to the downward operating function can be selectively assigned to the downward button 64. For example, in initial setting, a reproduction function is assigned to the downward button 64. Reproduction of a latest captured image is started when the downward button 64 is operated.

The rightward button 66 is operated when a screen rightward operation is performed on an image, a menu screen, or the like displayed on the display unit 61. That is, the rightward operating function is assigned to the rightward button 66.

Specifically, the rightward button 66 is operated, for example, when a cursor or the like is moved in the screen rightward direction on a menu screen or when a scrolling operation of displaying an area close to a right end of an image acquired by imaging, for example.

Further, a function display function (hereinafter referred to as a Fn function) of displaying a function setting screen in addition to the rightward operating function is assigned to the rightward button 66. That is, for example, when the rightward button 66 is operated at the time of capturing an image, or the like, a function setting screen is displayed on the display unit 61.

Characters "Fn" indicating the Fn function are printed on the right of the rightward button 66 in the drawing, that is, in a part indicated by an arrow W12, on the rear surface of the imaging device 11. A user can instantaneously understand that the Fn function is assigned to the rightward button 66 by seeing the printed characters.

Incidentally, an example in which characters indicating functions assigned in advance to the buttons, such as characters "DISP" and "Fn," are printed in the vicinity of the buttons is described, but symbols indicating functions or the like may be printed.

The leftward button 65 is disposed on the left of the rightward button 66 in the drawing. The leftward button 65 is operated when a screen leftward operation is performed. That is, a leftward operating function is assigned to the leftward button 65.

Further, an arbitrary function among a plurality of predetermined functions in addition to the leftward operating function can be selectively assigned to the leftward button 65. For example, in initial setting, a white balance (WB) setting function is assigned to the leftward button 65. When the leftward button 65 is operated at the time of capturing an image or the like, a WB setting screen for selecting an imaging environment serving as a premise for white balance adjustment is displayed on the display unit 61.

In this way, the upward button 63, the downward button 64, the leftward button 65, and the rightward button 66 are associated with upward, downward, leftward, and rightward directions, and functions of performing an operation in the directions associated with the buttons (keys) and functions associated with no direction are assigned to thereto. That is, a function associated with a direction and a function associated with no direction are assigned in advance to the buttons which are operation units associated with a direction.

In the following description, the upward button 63, the downward button 64, the leftward button 65, and the rightward button 66 are also referred to as direction keys when they are not particularly distinguished from each other. In the example illustrated in Fig. 3, an arrow indicating a direction associated with each direction key is printed on the direction key. Further, at least one function has only to be assigned to each direction key. That is, one or more functions can be assigned to each direction key.

Furthermore, a menu button 67 is disposed on the left of the leftward button 65 in the drawing. The menu button 67 is operated when a menu screen is displayed on the display unit 61. That is, a menu display function of displaying a menu screen is assigned to the menu button 67.

A cover 68 is attached to the left of the display unit 61 in the drawing on the rear surface of the imaging device 11. The cover 68 can be detached from the imaging device 11 by operating a lock button 69.

As indicated by an arrow Q11, various terminals or slots are provided below the cover 68 in the main body of the imaging device 11. Here, the diagram indicated by the arrow Q11 illustrates a state in which the cover 68 is detached from the left part of the rear surface of the main body of the imaging device 11.

In this example, a high-definition multimedia interface (HDMI) (registered trademark) terminal 70 and a universal serial bus (USB) (registered trademark) terminal 71 are provided as input and output terminals for external connection below the cover 68 of the main body of the imaging device 11.

Further, a terminal 72 for attachment of a microphone, a slot 73 which is an insertion hole of a removable recording medium detachable from the imaging device 11, and a medium access lamp 74 for showing an access to the removable recording medium or the like are provided below the cover 68 of the main body of the imaging device 11.

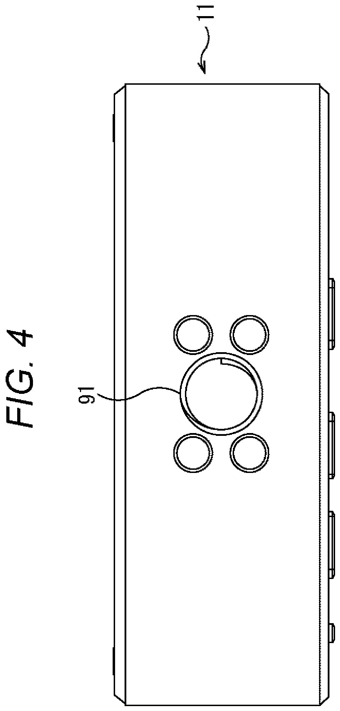

Furthermore, a socket 91 which is a fixing portion for fixing the imaging device 11 to a tripod or the like is provided on the bottom surface of the imaging device 11, for example, as illustrated in Fig. 4. When the imaging device 11 is fixed to a ceiling, a wall, or the like using the socket 91, capturing an image can be performed in a state in which the imaging device 11 is inverted or rotated by 90 degrees, for example.

### <Example of Functional Configuration of Imaging Device>

An example of a functional configuration of the imaging device 11 will be described below.

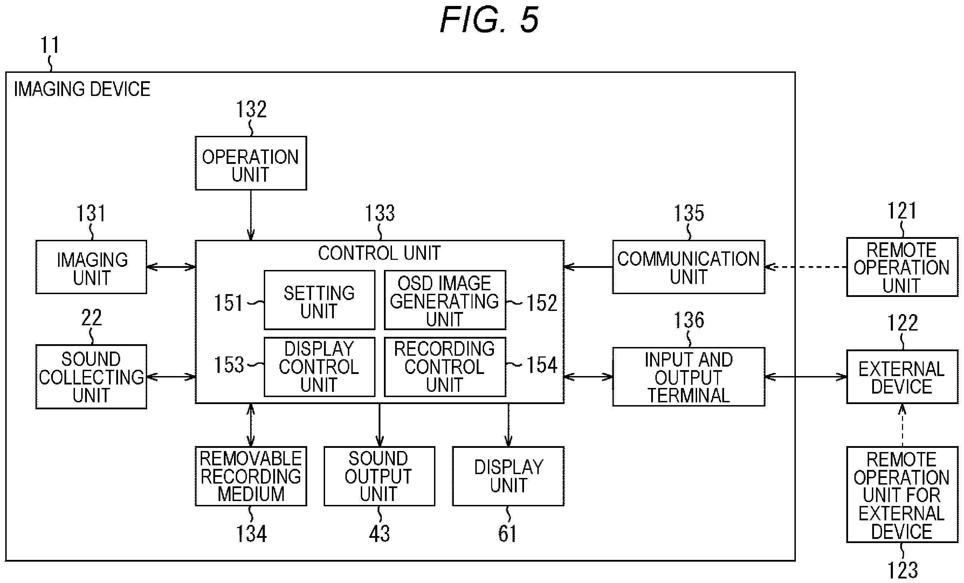

Fig. 5 is a diagram illustrating an example of a functional configuration of the imaging device 11. Incidentally, elements in Fig. 5 corresponding to those in Figs. 1 to 4 will be referenced by the same reference signs and description thereof will be appropriately skipped.

In the example illustrated in Fig. 5, the imaging device 11 can be remotely operated with a remote operation unit 121, and an external device 122 such as a recorder with a display or a display is connected to the imaging device 11. In the following description, it is assumed that the external device 122 is a recorder with a display. In addition, the external device 122 and the imaging device 11 can be remotely operated by an external-device remote operation unit 123 dedicated for the external device 122.

The imaging device 11 includes an imaging unit 131, a sound collecting unit 22, an operation unit 132, a control unit 133, a removable recording medium 134, a sound output unit 43, a display unit 61, a communication unit 135, and an input and output terminal 136.

The imaging unit 131 includes the lens 21 illustrated in Fig. 1, an imaging element, or the like, captures an image by receiving light from a subject and photoelectrically converting the received light under the control of the control unit 133, and supplies image data of the acquired image to the control unit 133. Incidentally, in the following description, an image acquired by the imaging unit 131 is also particularly referred to as a captured image.

The sound collecting unit 22 collects ambient sound under the control of the control unit 133 and supplies sound data of the collected sound which is acquire as a result to the control unit 133.

The operation unit 132 includes, for example, the power supply button 41 and the shutter button 42 which are illustrated in Fig. 2, the upward button 63 to the menu button 67 which are illustrated in Fig. 3, a touch panel which is disposed to overlap the display unit 61, and the like, and supplies a signal corresponding to a user's operation to the control unit 133.

The control unit 133 controls the whole operations of the imaging device 11. The control unit 133 includes a setting unit 151, an on screen-display (OSD) image generating unit 152, a display control unit 153, and a recording control unit 154.

The setting unit 151 performs various settings on the basis of a signal from the operation unit 132. The OSD image generating unit 152 generates an OSD image.

Examples of an OSD image include images indicating marks or images indicating various settings or states of the imaging device 11, such as a mark indicating an imaging mode, an image indicating an amount of residual power of a battery, a mark indicating white balance setting, and the like.

The display control unit 153 controls display of various images on the display unit 61 or the external device 122. The recording control unit 154 controls recording of image data acquired by imaging or sound data acquired by sound collection on the removable recording medium 134 or the external device 122.

The removable recording medium 134 is a recording medium that can be attached to and detached from the imaging device 11, and is attached to the imaging device 11 when it is inserted into the slot 73 illustrated in Fig. 3. The removable recording medium 134 records image data or sound data supplied from the control unit 133 and supplies image data or sound data recorded thereon to the control unit 133 if necessary.

The communication unit 135 receives a command transmitted from the remote operation unit 121 by wireless or wired communication and supplies the received command to the control unit 133.

For example, the remote operation unit 121 is a remote commander dedicated for the imaging device 11 or for a general purpose that transmits a command by wireless communication using infrared light or the like or wired communication using the terminal 71 or the like, and may be embodied by a smartphone or the like.

The input and output terminal 136 is, for example, an input and output unit that includes the terminal 70 or the terminal 71 illustrated in Fig. 3, or the like, and communicates with the external device 122.

For example, the input and output terminal 136 outputs image data or sound data supplied from the control unit 133 to the external device 122 or outputs a command or the like supplied from the external device 122 to the control unit 133.

### <Flip Setting>

Incidentally, as described above, the imaging device 11 may be used in a state in which a user takes it with a hand or may be used in a state in which it is fixed to a ceiling, a wall, or the like.

For example, when the imaging device 11 is used in a state in which it is fixed inversely, a captured image is recorded in a vertically inverted state and right and left channels of collected sound are recorded in an exchanged state .

Therefore, a flip function for prohibiting vertical inversion of an image even when the imaging device 11 is used in an inverted state is provided in the imaging device 11. The flip function is a function of vertically inverting a display direction of an image such as a captured image, a reproduced image, or a menu screen to a predetermined direction, that is, the vertical direction herein. Further, in this embodiment, even when the imaging device 11 is used in an inverted state, the right and left channels of sound are not inverted by the flip function.

Here, as for a captured image acquired by the imaging unit 131, an image for ascertaining an angle of view which is displayed on the display unit 61 during imaging is referred to as a through-image, and a recording image which is to be recorded on the removable recording medium 134 or the like is referred to as a recording image. Further, sound acquired by the sound collecting unit 22 is also referred to as collected sound, and recording sound which is to be recorded on the removable recording medium 134 or the like is also referred to as recording sound.

For example, when flip setting is turned off, that is, when the flip function is set to OFF, it is assumed that the imaging device 11 is used in a non-inverted state, that is, in a state in which the bottom surface of the imaging device 11 faces the lower side (the ground surface side) in the vertical direction.

Accordingly, in the state in which flip setting is turned off, a captured image and collected sound become a recording image and recording sound without any change.

Further, although details thereof will be described later, a display screen such as an OSD image or a menu screen is also displayed such that the top surface of the imaging device 11 faces the screen upward direction in the state in which flip setting is turned off.

Incidentally, in the following description, images (screens) which are displayed on the display unit 61 such as a recording image, an OSD image, and a menu image are also simply referred to as a display screen when they are not particularly distinguished. Here, a through-image is not included in (is excluded from) the display screen.

Further, in the following description, a display state in which a top end of a display screen, that is, an end in the screen upward direction, is located on the top surface side of the display unit 61 when the display screen is displayed on the display unit 61 is referred to as a normal display state. In the normal display state, characters and the like are displayed on the display unit 61 erectly to a user who uses the imaging device 11 in a state in which the top surface thereof is located on the upper side when seen from the user.

On the other hand, in a state in which flip setting is turned on, that is, the flip function is set to ON, a captured image is vertically inverted, that is, the captured image is rotated by 180 degrees, to become a recording image and the right and left channels of collected sound are exchanged to become recording sound.

Further, although details will be described later, a display screen such as an OSD image or a menu screen is vertically inverted and displayed such that the bottom surface of the imaging device 11 faces the screen upward direction in a state in which flip setting is turned on. That is, the display direction of the display screen is vertically inverted.

Incidentally, in the following description, a display state in which a top end of a display screen, that is, an end in the screen upward direction, is located on the bottom surface side of the display unit 61 when the display screen is displayed on the display unit 61 is referred to as an inverted display state.

In the inverted display state, characters and the like are displayed on the display unit 61 erectly to a user who uses the imaging device 11 in a state in which the bottom surface thereof is located on the upper side when seen from the user. In other words, in the inverted display state, characters and the like are displayed on the display unit 61 in the vertically inverted state to a user who uses the imaging device 11 in a state in which the top surface thereof is located on the upper side when seen from the user.

Furthermore, in the state in which flip setting is turned on, behavior of the direction keys of the imaging device 11 also varies. That is, functions assigned to two direction keys associated with different directions are exchanged between the two direction keys.

When the flip function is set to ON in this way, vertical inversion of a captured image at the time of generating a recording image, exchange of the right and left channels of collected sound at the time of generating recording sound, vertically inverted display of a display screen, and exchange of assigned functions between the direction keys are performed, but at least one thereof may be performed. For example, when the flip function is set to ON, at least vertical inversion of a display direction of an image such as vertical inversion of a capture image at the time of generating a recording image or vertically inverted display of a display screen may be performed. In this case, exchange of the right and left channels of collected sound at the time of generating recording sound or exchange of assigned functions between the direction keys may be performed or may not be performed.

### <Operation in Flip Setting>

Flip setting will be described below in more details.

First, an operation when the flip function is set to ON or OFF will be described.

A user can perform a setting operation of setting flip setting to ON or OFF by operating the menu button 67 disposed on the rear surface of the imaging device 11 or the like to display a menu screen on the display unit 61.

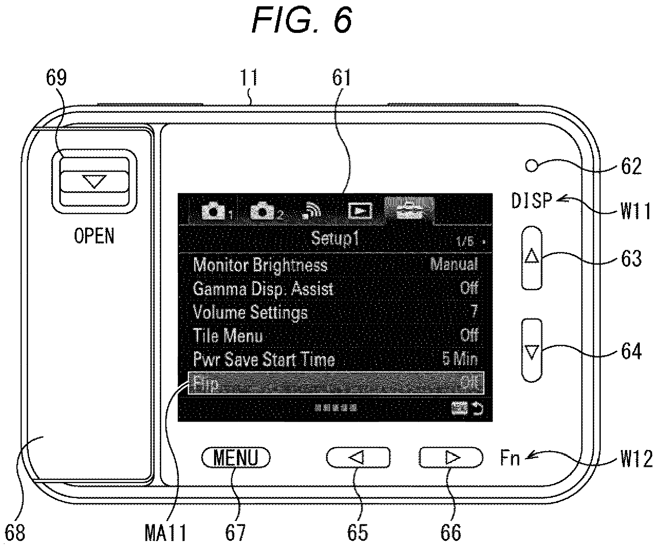

For example, when the menu button 67 is operated in the state in which flip setting is turned off, the display control unit 153 supplies data of a menu screen to the display unit 61 in response to a signal supplied from the menu button 67 serving as the operation unit 132 and displays the menu screen illustrated in Fig. 6.

In the example illustrated in Fig. 6, a plurality of menu items including a menu item MA11 in which characters "Flip" are displayed are arranged (displayed) in parallel on the menu screen. The menu item MA11 is a menu item for performing flip setting, and characters "Off" displayed in the menu item MA11 indicate that current flip setting is turned off. Accordingly, in the example illustrated in Fig. 6, the display state of the menu screen is the normal display state.

Further, in this example, the menu item MA11 is emphasized, that is, is in a display state different from that of other menu items, and the menu item MA11 is in the selected state.

In this state, for example, it is assumed that the user operates the shutter button 42 or the like disposed on the top surface of the imaging device 11 and instructs to determine selection of the menu item MA11.

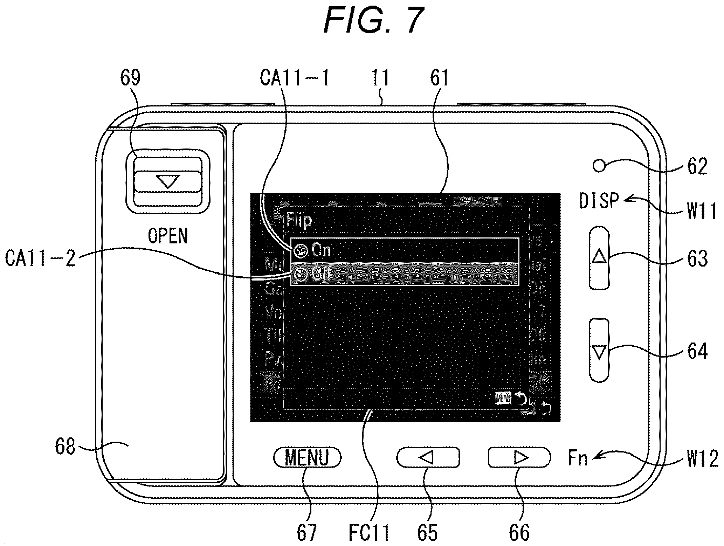

Then, the display control unit 153 supplies data of a flip setting screen to the display unit 61 in response to a signal supplied from the shutter button 42 or the like serving as the operation unit 132 and displays, for example, the flip setting screen illustrated in Fig. 7.

In the example illustrated in Fig. 7, since a flip setting screen FC11 for performing flip setting is displayed to overlap the menu screen illustrated in Fig. 6 and flip setting is turned off, the flip setting screen FC11 is also displayed in the normal display state.

A setting item CA11-1 for setting the flip function to ON and a setting item CA11-2 for setting the flip function to OFF are displayed on the flip setting screen FC11. Particularly, characters "On" are displayed in the setting item CA11-1, and characters "Off" are displayed in the setting item CA11-2. Incidentally, in the following description, the setting item CA11-1 and the setting item CA11-2 are also simply referred to as a setting item CA11 when they are not particularly distinguished from each other.

When the flip setting screen FC11 is displayed, the user performs flip setting by operating the direction keys, the shutter button 42, or the like to select the setting item CA11 or to determine selection of the setting item CA11 in the selected state.

In the example illustrated in Fig. 7, the setting item CA11-2 for turning off flip setting is emphasized and selected. When an operation of determining selection of the setting item CA11-2 is performed in this state, the setting unit 151 turns off the flip setting on the basis of a signal supplied from the operation unit 132 and records flip setting information indicating the setting result.

Incidentally, more specifically, the setting unit 151 records flip setting information and updates the flip setting information by setting the recorded flip setting information to information indicating that the flip function is set to OFF when selection of the setting item CA11-2 is determined.

When flip setting is turned off in this way, the display control unit 153 then returns, for example, the display of the display unit 61 to the menu screen illustrated in Fig. 6 or returns the display to a state before the menu screen is displayed, for example, a state in which a through-image is displayed.

On the other hand, when the setting item CA11-1 for turning on flip setting is selected and an operation of determining the selection of the setting item CA11-1 is performed by the user, the setting unit 151 turns on flip setting on the basis of a signal supplied from the operation unit 132 and records flip setting information indicating the setting result. That is, the flip setting information is updated with information indicating that the flip function is set to ON.

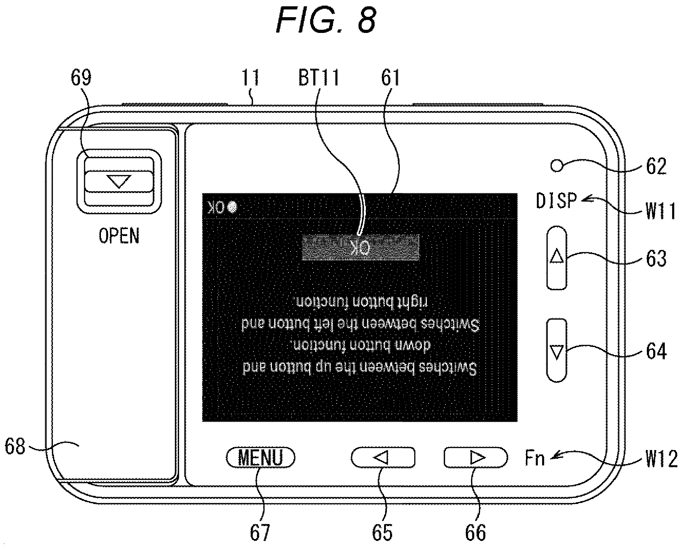

Further, when flip setting is turned on, the display control unit 153 controls the display unit 61 immediately thereafter such that an explanation screen which is a screen associated with flip setting illustrated in Fig. 8, that is, a screen associated with setting of the flip function, is displayed, for example.

In this example, since flip setting is turned on, the explanation screen is displayed in the inverted display state . That is, the explanation screen is displayed in the vertically inverted state on the display unit 61 such that the top end of the explanation screen faces the bottom surface of the imaging device 11.

Explanation of the flip function is displayed on the explanation screen. Specifically, characters "Switches between the up button and down button function." and characters "Switches between the left button and right button function." which indicate explanation of behavior of the operation unit 132 when flip setting is turned on, particularly, behavior of the direction keys serving as the operation unit 132 are displayed on the explanation screen.

That is, explanation indicating that behavior (functions) of buttons associated with opposite directions such as the upward button 63 and the downward button 64 among the direction keys is displayed on the explanation screen. Similarly, explanation indicating that behavior (functions) of buttons associated with opposite directions such as the leftward button 65 and the rightward button 66 among the direction keys is also displayed on the explanation screen.

Incidentally, details of behavior of the direction keys or the like based on flip setting will be described later. Further, a case where explanation of behavior of the direction keys is displayed has been described herein as an example of the explanation screen. However, the explanation screen is not particularly limited as long as explanation indicating behavior of the imaging device 11 or the like including a display state when flip setting is turned on such as explanation indicating that the display is vertically inverted or explanation of behavior of a remote operation unit is displayed.

As described above, in the imaging device 11, immediately after flip setting is turned on, an explanation screen on which explanation of behavior of the imaging device 11 is displayed when flip setting is turned on is displayed in the inverted display state. Accordingly, a user can be notified that flip setting is turned on and can be caused to intuitively and easily understand behavior after the setting and it is thus possible to improve usability of the imaging device 11. Particularly, by displaying the explanation screen in the inverted display state, a user can easily understand that the display of the display unit 61 is vertically inverted.

Further, a button BT11 which is operated when a user has ascertained the explanation details of the explanation screen is also displayed in the explanation screen illustrated in Fig. 8, and characters "OK" are displayed in the button BT11.

By providing the button BT11 on the explanation screen, a user can be prompted to ascertain that flip setting is turned on. Accordingly, in this example, the explanation screen also serves as an ascertainment screen for performing ascertainment of flip setting, that is, ascertainment of setting of the flip function.

When the button BT11 is operated by a user, the display of the display unit 61 is then returned to, for example, the menu screen illustrated in Fig. 6 or is returned to a state before the menu screen is displayed, for example, a state in which a through-image is displayed.

Furthermore, an example in which the button BT11 which is an OK button for ascertaining that flip setting is turned on is provided on the explanation screen has been described herein, but a cancellation button for cancelling the operation of turning on flip setting in addition to the button BT11 may be also provided.

In this case, a user operates the cancellation button on the explanation screen to invalidate the selecting operation of turning on flip setting which has been performed by the user, by operating the direction keys or the like serving as the operation unit 132.

When the cancellation button is operated by a user in a state in which the explanation screen (the ascertainment screen) is displayed and it is instructed to cancel the setting of turning on the flip function, the setting unit 151 cancels the setting of the flip function which has been performed immediately before the explanation screen is displayed on the basis of a signal from the operation unit 132. Further, the display control unit 153 controls the display unit 61 such that the display of the display unit 61 is returned to the flip setting screen FC11 illustrated in Fig. 7. Then, when flip setting is newly performed, the setting unit 151 records flip setting information indicating the new setting result.

When setting of turning on the flip function which has been performed immediately before can be cancelled on the explanation screen of flip setting in this way, it is possible to further improve usability.

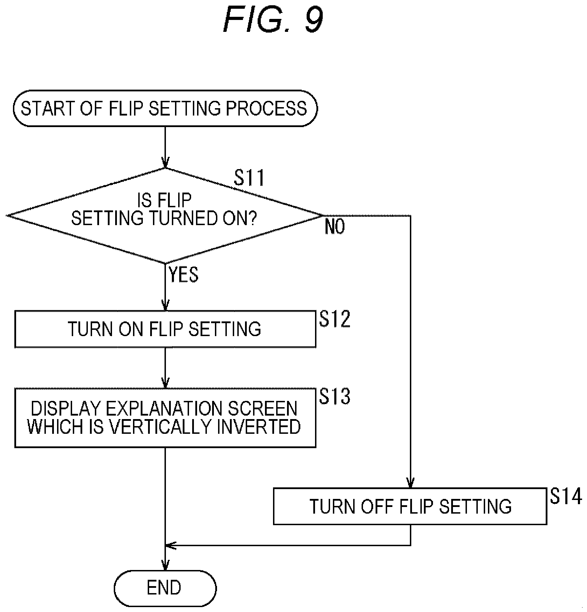

A specific operation of the imaging device 11 when the above-mentioned flip setting is performed will be described below. That is, a flip setting process which is performed by the imaging device 11 will be described below with reference to the flowchart illustrated in Fig. 9. This flip setting process is started when the flip setting screen FC11 illustrated in Fig. 7 is displayed on the display unit 61.

In Step S11, the setting unit 151 determines whether or not to turn on flip setting on the basis of a signal from the operation unit 132.

For example, when the operation unit 132 is operated by a user and selection of the setting item CA11-1 on the flip setting screen FC11 is determined, it is determined that flip setting is turned on. On the other hand, when selection of the setting item CA11-2 on the flip setting screen FC11 is determined, it is determined that flip setting is not to be turned on, that is, to be turned off.

When it is determined in Step S11 that flip setting is to be turned on, that is, when an instruction to turn on the flip function is received, the setting unit 151 turns on flip setting on the basis of a signal supplied from the operation unit 132 in response to an operation of determining selection of the setting item CA11-1 by a user in Step S12.

Specifically, the flip setting information recorded by the setting unit 151 is updated with information indicating that flip setting is turned on. In other words, the setting unit 151 generates flip setting information indicating that flip setting is turned on and records the generated flip setting information.

In Step S13, the display control unit 153 supplies data of the explanation screen to the display unit 61, controls the display unit 61 on the basis of the flip setting information, which is recorded in the setting unit 151, indicating that flip setting is turned on such that the vertically inverted explanation screen is displayed on the display unit 61. Accordingly, for example, as illustrated in Fig. 8, the explanation screen is displayed in the inverted display state on the display unit 61.

When the explanation screen is displayed, the flip setting process ends. In this case, when the button BT11 is operated by a user, the display of the display unit 61 is returned to the menu screen or is returned to display of a through-image .

On the other hand, when it is determined in Step S11 that flip setting is not to be turned on, that is, to be turned off, the setting unit 151 turns off flip setting on the basis of a signal supplied from the operation unit 132 in response to an operation of determining selection of the setting item CA11-2 by a user in Step S14.

Specifically, the flip setting information recorded by the setting unit 151 is updated with information indicating that flip setting is turned off. In other words, the setting unit 151 generates flip setting information indicating that flip setting is turned off and records the generated flip setting information.

When flip setting is turned off, the flip setting process ends thereafter. In this case, the display of the display unit 61 is returned to, for example, the menu screen or is returned to display of a through-image. That is, when flip setting is turned off, the display control unit 153 does not display a screen associated with setting of the flip function such as the explanation screen illustrated in Fig. 8, for example.

When the flip setting information is recorded and flip setting is turned on in response to a user's operation of the flip setting screen in this way, the imaging device 11 displays the explanation screen in the inverted display state immediately thereafter. Accordingly, the user can be caused to easily understand whether flip setting is turned on or what behavior is performed when flip setting is turned on and it is thus possible to improve usability of the imaging device 11.

### <Capturing of Moving Image>

Operations at the time of capturing an image when flip setting is turned on and off will be described below.

For example, when a moving image is captured in a state in which flip setting is turned off, the captured image and the collected sound become a recording image and recording sound without any change. Further, display in the normal display state is performed on the display unit 61.

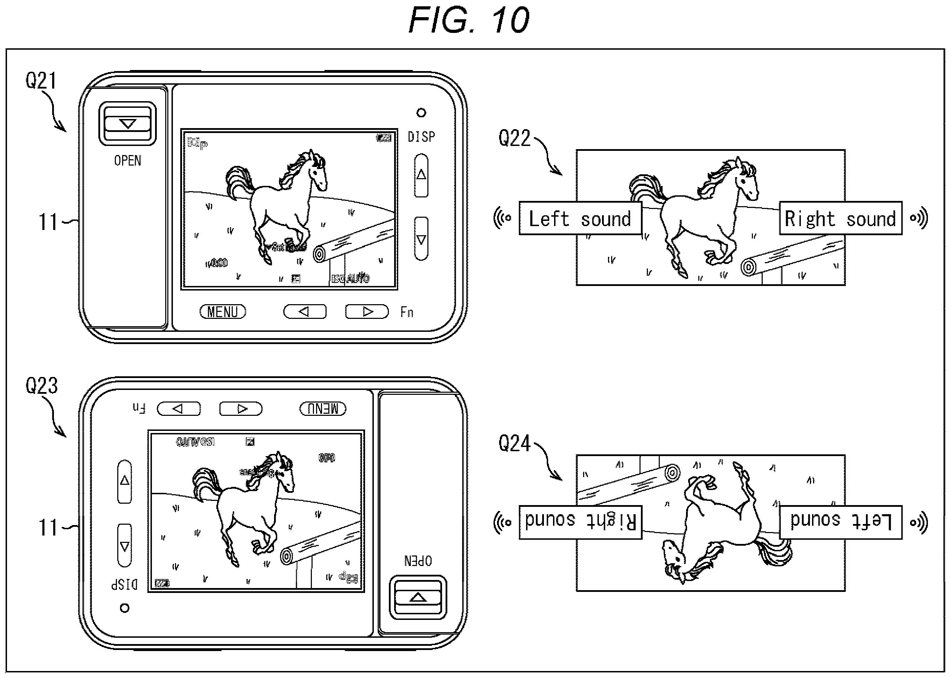

Accordingly, for example, when imaging is performed in a state in which the bottom surface of the imaging device 11 faces the ground surface as indicated by an arrow Q21 in Fig. 10 (hereinafter also referred to as a normal use state), an OSD image or a through-image displayed on the display unit 61 is displayed erectly to a user.

Incidentally, when a user uses the imaging device 11 in the normal use state, it is assumed that the user sees the display unit 61 in a state in which the top surface thereof is located on the upper side of the imaging device 11 when seen from the user and the bottom surface thereof is located on the lower side of the imaging device 11 when seen from the user. This is the same in the following description.

Further, when a user uses the imaging device 11 in a state in which the imaging device 11 is vertically inverted (hereinafter also referred to as an inverted use state), it is assumed that the user sees the display unit 61 in a state in which the bottom surface thereof is located on the upper side of the imaging device 11 when seen from the user and the top surface thereof is located on the lower side of the imaging device 11 when seen from the user.

Furthermore, when it is mentioned that an OSD image or a through-image is displayed erectly to a user, it means that characters on the OSD image are neither inverted nor laid down but are displayed erectly to the user in the same way as the state in which a user generally sees the characters and a subject on the through-image is also displayed to the user in the same way as the user actually sees the subject. In the following description, when it is mentioned that something is seen erectly by a user, it means that an image or the like is seen from the user in this state.

When flip setting is turned off and imaging is performed in the normal use state as indicated by the arrow Q21, a recording image is also recorded in the similar state to the user actually sees a subject as indicated by an arrow Q22 and recording sound is also recorded in a state in which the right and left channels are right.

Further, for example, when flip setting is turned off and imaging is performed in the inverted use state as indicated by an arrow Q23, that is, in a state in which the imaging device 11 is vertically inverted, a subject is displayed on the through-image as if the user sees an actual subject, but the OSD image is displayed in the vertically inverted state to the user.

Furthermore, when flip setting is turned off and imaging is performed in the inverted use state as indicated by the arrow Q23, the recording image is recorded in the vertically inverted state unlike a subject which is actually seen by the user as indicated by an arrow Q24 and the recording sound is also recorded in a state in which the right and left channels are inverted unlike actual sound.

In this way, when flip setting is turned off, the normal use state is a right use state and the inverted use state is a wrong use state. That is, when flip setting is turned off, use of the imaging device 11 in the normal use state is assumed and use thereof in the inverted use state is not assumed.

On the other hand, when a moving image is captured in a state in which flip setting is turned on, a captured image is vertically inverted to be a recording image, and the right and left channels of collected sound are exchanged to be recording sound. Further, display in the inverted display state is performed on the display unit 61. Here, a through-image is not displayed in the inverted display state and is displayed in the normal display state.

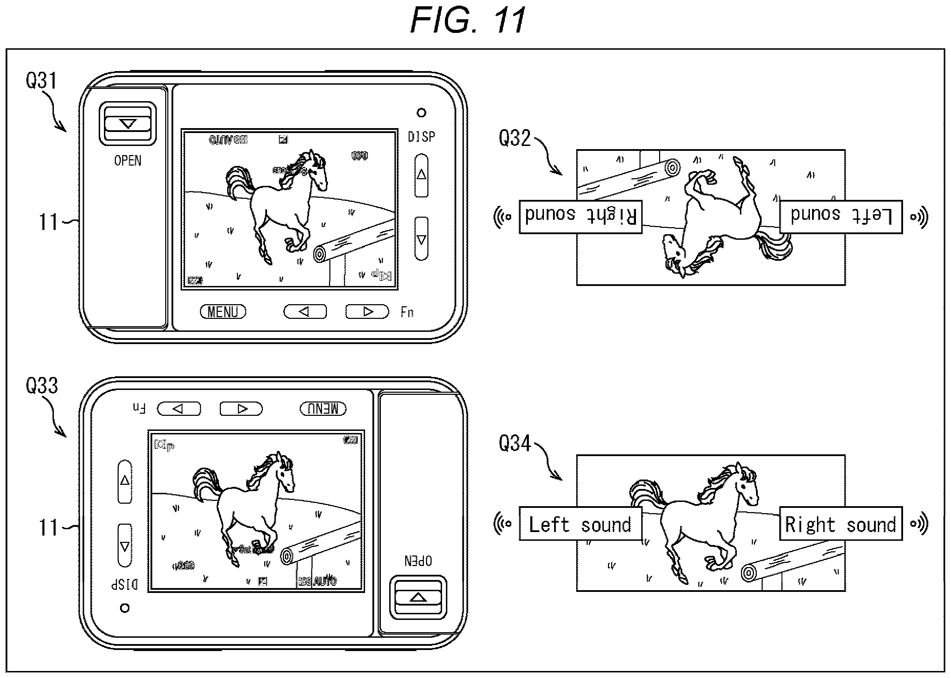

Accordingly, when flip setting is turned on and imaging is performed by the imaging device 11 in the normal use state, for example, as indicated by an arrow Q31 in Fig. 11, a subject is displayed on a through-image in the same way as a user sees the actual subject, but an OSD image is displayed in the vertically inverted state to the user.

When flip setting is turned on and imaging is performed in the normal use state as indicated by the arrow Q31, the recording image is recorded in the vertically inverted state unlike a subject which is actually seen by the user as indicated by an arrow Q32 and the recording sound is also recorded in a state in which the right and left channels are inverted unlike actual sound.

Furthermore, for example, when flip setting is turned on and imaging is performed by the imaging device 11 in the inverted use state as indicated by an arrow Q33, an OSD image or a through-image displayed on the display unit 61 is seen erectly by a user.

When flip setting is turned on and imaging is performed in the inverted use state as indicated by the arrow Q33, the recording image is recorded in the similar state to the user actually sees a subject as indicated by an arrow Q34 and the recording sound is also recorded in a state in which the right and left channels thereof are right.

In this way, when flip setting is turned on, the inverted use state is a right use state and the normal use state is a wrong use state. That is, when flip setting is turned on, use of the imaging device 11 in the inverted use state is assumed and use thereof in the normal use state is not assumed.

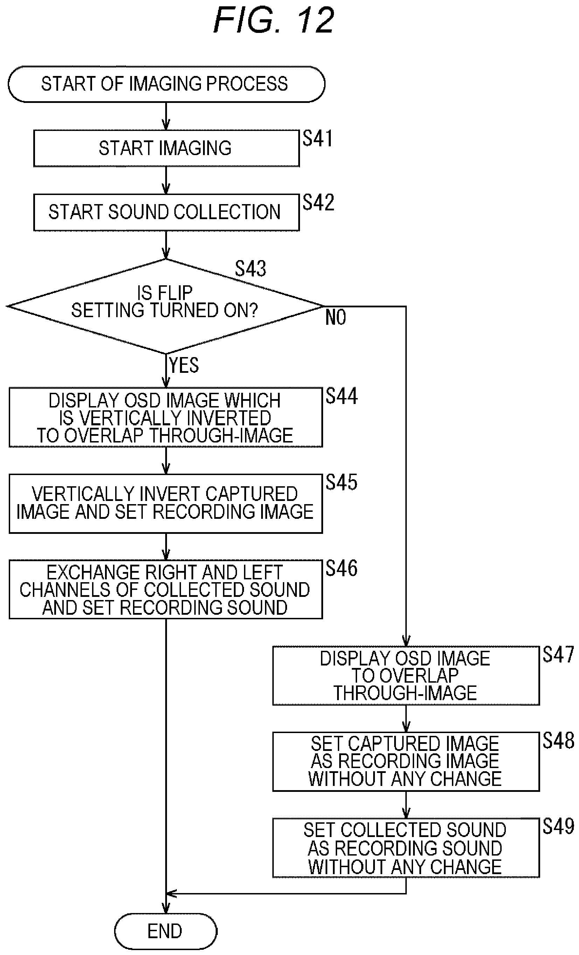

The above-mentioned operation of the imaging device 11 when a moving image is captured will be described below. That is, an imaging process using the imaging device 11 will be described below with reference to the flowchart illustrated in Fig. 12.

In Step S41, the imaging unit 131 starts capturing an image. That is, the imaging unit 131 captures an image of each frame by receiving light which is incident from a subject and performing photoelectric conversion and sequentially supplies the acquired image data to the control unit 133.

In Step S42, the sound collecting unit 22 starts collection of ambient sound and sequentially supplies sound data of the collected sound to the control unit 133.

In Step S43, the control unit 133 determines whether or not flip setting is turned on. For example, when flip setting information recorded in the setting unit 151 is information indicating that flip setting is turned on, it is determined that flip setting is turned on.

When it is determined in Step S43 that flip setting is turned on, the display control unit 153 displays a vertically inverted OSD image to overlap a through-image in Step S44.

That is, the display control unit 153 supplies image data of the captured image acquired by the imaging unit 131 as image data of a through-image to the display unit 61 without any change and displays the through-image on the display unit 61.

Further, the display control unit 153 generates image data in which an OSD image is displayed in the vertically inverted state, that is, an OSD image is displayed in the inverted display state, on the basis of the image data of the OSD image generated by the OSD image generating unit 152 in accordance with various settings, and sets the generated image data as image data of a final OSD image. Then, the display control unit 153 supplies the generated image data of the OSD image to the display unit 61 and controls the display unit 61 such that the OSD image in the inverted display state is displayed to overlap the through-image.

Accordingly, for example, the through-image and the OSD image which are indicated by an arrow Q31 or indicated by an arrow Q33 in Fig. 11 are displayed on the display unit 61.

In Step S45, the recording control unit 154 vertically inverts the captured image and sets the inverted captured image as a recording image.

That is, the recording control unit 154 generates image data for displaying a captured image which is displayed in the inverted display state, that is, an image which is displayed in the vertically inverted state, as image data for a recording image on the basis of the image data of the captured image supplied from the imaging unit 131.

In Step S46, the recording control unit 154 exchanges the right and left channels of collected sound and sets the exchanged sound as recording sound.

That is, the recording control unit 154 generates sound data in which the right and left channels of sound data are exchanged on the basis of the sound data of the collected sound supplied from the sound collecting unit 22 and sets the generated sound data as sound data for recording sound.

Through the above-mentioned processes, image data of a recording image and sound data of recording sound for reproducing a moving image are acquired, for example, as indicated by an arrow Q32 or an arrow Q34 in Fig. 11.

When the image data of a recording image and the sound data of recording sound are acquired, for example, the recording control unit 154 supplies the image data and the sound data to the removable recording medium 134 and records the image data and the sound data therein. Further, for example, the image data of a recording image and the sound data of recording sound may be supplied to the external device 122 via the input and output terminal 136 by the control unit 133.

Then, when the image data of a recording image and the sound data of recording sound are appropriately recorded or the like, the imaging process ends.

On the other hand, when it is determined in Step S43 that flip setting is not turned on, that is, when flip setting is turned off, the process flow transitions to Step S47.

In Step S47, the display control unit 153 displays an OSD image to overlap a through-image.

That is, the display control unit 153 supplies image data of the captured image acquired by the imaging unit 131 as image data of a through-image to the display unit 61 without any change and displays the through-image on the display unit 61.

Further, the display control unit 153 supplies the image data of an OSD image, which is generated by the OSD image generating unit 152 in accordance with various settings, to the display unit 61 without any change and controls the display unit 61 such that the OSD image in the normal display state is displayed to overlap the through-image.

Accordingly, for example, the through-image and the OSD image which are indicated by an arrow Q21 or indicated by an arrow Q23 in Fig. 10 are displayed on the display unit 61.

In Step S48, the recording control unit 154 sets the captured image as a recording image without any change. That is, the recording control unit 154 sets the image data of the captured image supplied from the imaging unit 131 as image data of a recording image without any change.

In Step S49, the recording control unit 154 sets the collected sound as recording sound without any change. That is, the recording control unit 154 sets the sound data of the collected sound supplied from the sound collecting unit 22 as sound data of recording sound without any change.

Through the above-mentioned processes, image data of a recording image and sound data of recording sound for reproducing a moving image are acquired, for example, as indicated by an arrow Q22 or an arrow Q24 in Fig. 10.

When the image data of a recording image and the sound data of recording sound are acquired, for example, the recording control unit 154 supplies the image data and the sound data to the removable recording medium 134 and records the image data and the sound data therein. Further, for example, the image data of a recording image and the sound data of recording sound may be supplied to the external device 122 via the input and output terminal 136 using the control unit 133.

Then, when the image data of a recording image and the sound data of recording sound are appropriately recorded or the like, the imaging process ends.

In this way, the imaging device 11 generates the recording image and the recording sound depending on whether flip setting is turned on or off. Particularly, by vertically inverting the captured image to be a recording image and exchanging the right and left channels of the collected sound to be recording sound when flip setting is turned on, it is possible to acquire a recording image and recording sound which are appropriately reproduced even when the imaging device 11 is used in the inverted use state.

### <Output of Image or the like to Outside>

Further, when an image or sound acquired by the imaging device 11 is output to the external device 122, outputting of an image or sound is controlled such that various images such as an OSD image are displayed in the normal display state and sound is reproduced rightly without depending on whether flip setting is turned on or off. This is, for example, because a display of the external device 122 is not often used in the vertically inverted state.

Therefore, when an OSD image or the menu screen is displayed on the external device 122, the imaging device 11 outputs data such as the OSD image or the menu screen to the external device 122 without any change such that they are displayed in the normal display sate. Further, the imaging device 11 outputs the image data of a recording image or the sound data of recording sound to the external device 122 without any change such that a moving image is reproduced rightly.

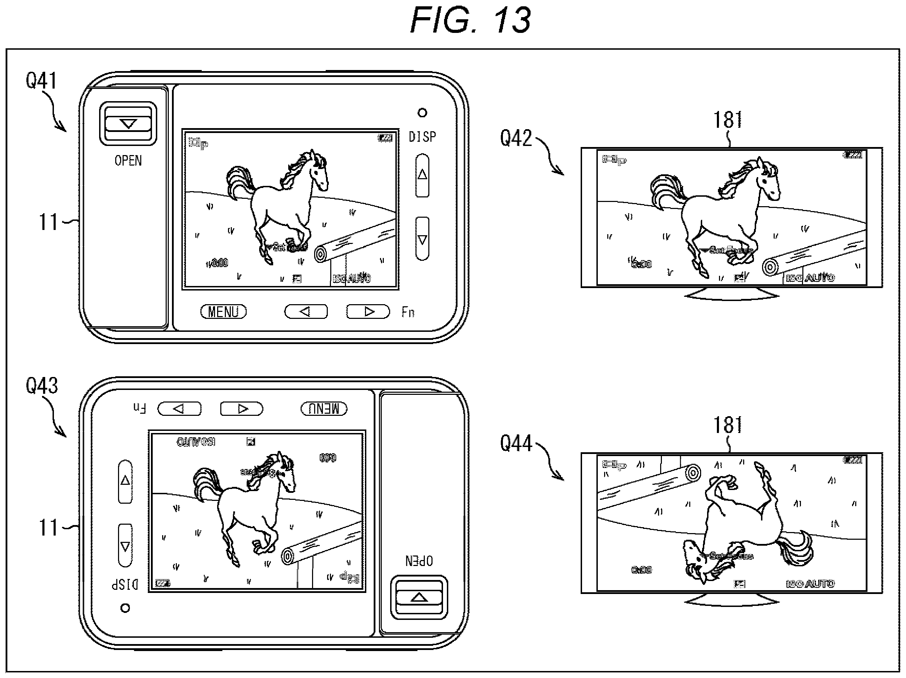

Accordingly, for example, when flip setting is turned off as indicated by an arrow Q41 in Fig. 13, it is assumed that capturing a moving image is performed in the normal use state and the acquired recording image and recording sound and the OSD image are output to the external device 122 such that they are displayed thereon.

In this case, as indicated by the arrow Q41, the OSD image or the through-image in the imaging device 11 is seen erectly by a user.

Further, in this case, as indicated by an arrow Q42, the OSD image and the recording image in the display unit 181 which is a display constituting the external device 122 is seen erectly by a user. That is, in the recording image, a subject is displayed in the same way as a user actually sees the subject.

On the other hand, for example, when flip setting is turned off as indicated by an arrow Q43, it is assumed that a moving image is captured in the inverted use state and the acquired recording image and recording sound and the OSD image are output to the external device 122 such that they are displayed thereon.

In this case, as indicated by the arrow Q43, a subject on a through-image in the imaging device 11 is displayed in the same way as a user actually sees the subject, but the OSD image is displayed in the vertically inverted state to the user.

Further, in this case, as indicated by an arrow Q44, the OSD image on the display unit 181 of the external device 122 is displayed erectly to a user, but the subject in the recording image is displayed in the vertically inverted state unlike the subject which is actually seen by the user.

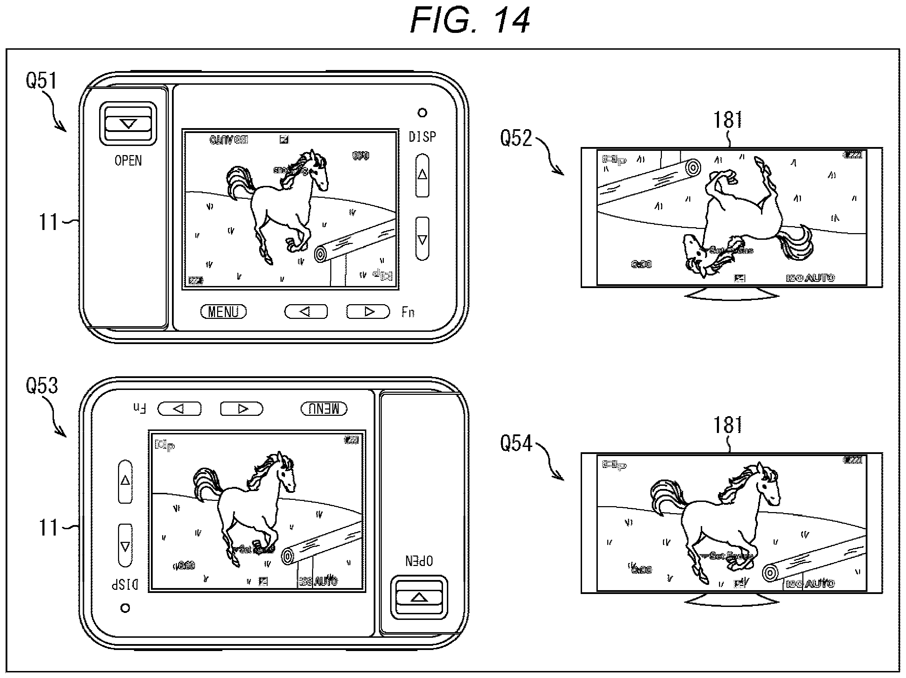

On the other hand, for example, when flip setting is turned on as indicated by an arrow Q51 in Fig. 14, it is assumed that a moving image is captured in the normal use state and the acquired recording image and recording sound and the OSD image are output to the external device 122 such that they are displayed thereon.

Further, in this case, as indicated by the arrow Q51, a subject on a through-image in the imaging device 11 is displayed in the same way as a user actually sees the subject, but the OSD image is displayed in the vertically inverted state to the user.

Further, in this case, as indicated by an arrow Q52, the OSD image in the display unit 181 of the external device 122 is displayed erectly to the user, but a subject in the recording image is displayed in the vertical inverted state unlike the subject which is actually seen by the user.

On the other hand, for example, when flip setting is turned on as indicated by an arrow Q53, it is assumed that a moving image is captured in the inverted use state and the acquired recording image and recording sound and the OSD image are output to the external device 122 such that they are displayed thereon.

In this case, as indicated by the arrow Q53, the OSD image or the through-image in the imaging device 11 is displayed erectly to the user.

Further, in this case, as indicated by an arrow Q54, the OSD image and the recording image in the display unit 181 of the external device 122 are displayed erectly to the user. That is, a subject in the recording image is displayed in the same way as the subject which is actually seen by the user.

In this way, when a recording image or recording sound is output to the external device 122 and imaging is performed in a right use state by the imaging device 11, the OSD image or the recording image in the external device 122 is displayed erectly and the recording sound can be rightly reproduced.

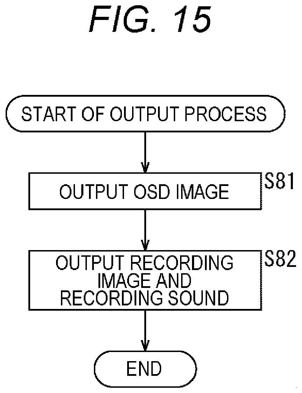

A process of causing the imaging device 11 to output an OSD image, a recording image, and recording sound to the external device 122 as described above will be described below. That is, an output process by the imaging device 11 will be described below with reference to the flowchart illustrated in Fig. 15.

This output process is performed, for example, when a moving image is being captured using the imaging device 11, that is, when the imaging process which has been described above with reference to Fig. 12 is being performed or when a moving image recorded on the removable recording medium 134 after the imaging, that is, the recording image and the recording sound, is reproduced by the external device 122, for example.

In Step S81, the control unit 133 outputs image data of an OSD image generated by the OSD image generating unit 152 to the external device 122 via the input and output terminal 136 without any change. Accordingly, the OSD image is displayed erectly on the external device 122.

In Step S82, the control unit 133 outputs image data of a recording image and sound data of recording sound to the external device 122 via the input and output terminal 136 without any change, and then ends the output process.

For example, when a moving image is being captured, the image data of a recording image and the sound data of recording sound which are acquired in the processes of Steps S45 and S46 in Fig. 12 or the processes of Steps S48 and S49 in Fig. 12 are output to the external device 122 in Step S82.

Further, for example, when a moving is not being captured, image data of a recording image and sound data of recording sound which are read from the removable recording medium 134 are output to the external device 122.

The image data of a recording image and the sound data of recording sound which are supplied to the external device 122 are recorded in the external device 122 or are used for a reproducing process in the external device 122. Accordingly, when a recording image is displayed on the display unit 181, a direction of a subject varies depending on the flip setting and the use state of the imaging device 11 as in the example indicated by the arrow Q42 or the arrow Q44 in Fig. 13 or the example indicated by the arrow Q52 or the arrow Q54 in Fig. 14, for example.

In this way, the imaging device 11 outputs the OSD image, the recording image, and the recording sound to the external device 122 without any change without depending on whether flip setting is turned on or off.

Accordingly, when an image is captured in the right use state by the imaging device 11 and an image of a subject is displayed on the display unit 181 of the external device 122 for ascertainment of an angle of view or the like, for example, during capturing a moving image, the OSD image or the recording image is displayed erectly on the external device 122 and the recording sound can also be rightly reproduced. Accordingly, it is possible to improve usability.

### <Operation of Direction Key>

Further, behavior of the direction keys serving as the operation unit 132 in the imaging device 11 varies depending on whether flip setting is turned on or off.

That is, in the state in which flip setting is turned off, the upward operating function and the DISP function are assigned to the upward button 63, and the downward operating function and another selected function are assigned to the downward button 64. Incidentally, in the following description, it is assumed that a reproduction function is assigned as another selected function to the downward button 64.

When flip setting is turned on in this state, the assigned functions of the upward button 63 and the downward button 64 associated with opposite directions are exchanged with each other.

That is, in the state in which flip setting is turned on, the downward operating function and the reproduction function are assigned to the upward button 63, and the upward operating function and the DISP function are assigned to the downward button 64.

In this case, for example, characters "DISP" are printed in the vicinity of the upward button 63 as illustrated in Fig. 3, but when the upward button 63 is operated, a process of realizing the reproduction function instead of the DISP function is performed. That is, when flip setting is turned on and the upward button 63 disposed around characters "DISP" is operated, the control unit 133 does not perform the process of realizing the DISP function indicated by the characters "DISP" but performs a process of realizing the reproduction function which is different from the process of realizing the DISP function.

Further, in the state in which flip setting is turned off, the leftward operating function and another selected function are assigned to the leftward button 65, and the rightward operating function and the Fn function are assigned to the rightward button 66. Incidentally, in the following description, it is assumed that a WB setting function is assigned as another selected function to the leftward button 65.

When flip setting is turned on in this state, the assigned functions of the leftward button 65 and the rightward button 66 associated with opposite directions are exchanged with each other.

That is, in the state in which flip setting is turned on, the rightward operating function and the Fn function are assigned to the leftward button 65, and the leftward operating function and the WB setting function are assigned to the rightward button 66.

In this case, for example, characters "Fn" are printed in the vicinity of the rightward button 66 as illustrated in Fig. 3, but when the rightward button 66 is operated, a process of realizing the WB setting function instead of the Fn function is performed. That is, even when the rightward button 66 is operated, the process of realizing the Fn function is not performed.

As for the direction keys associated with a direction, when flip setting is turned on, the assigned functions of the direction keys are exchanged between the direction keys associated with the opposite directions, that is, between a direction key associated with a predetermined direction and a direction key associated with a direction opposite to the predetermined direction. In other words, an assignment destination of a function is changed.

At this time, assignment destinations of the functions associated with no direction such as the DISP function, the Fn function, and the WB setting function in addition to the functions associated with a direction such as the upward operating function or the like, that is, the functions of realizing an operation associated with a direction are exchanged.

Furthermore, as for a button (a key) associated with no direction, the assigned function of the button is not changed without depending on whether flip setting is turned on or off.

For example, the menu button 67, the power supply button 41, and the shutter button 42 are buttons (the operation units) which are not associated with a direction, and the assigned functions of these buttons are not exchanged without depending on whether flip setting is turned on or off. That is, an assigned function of an operation unit which is not associated with a direction such as the menu button 67 is not changed.

Accordingly, a menu display function is assigned to the menu button 67, for example, without depending on whether flip setting is turned on or off.

As described above, by exchanging the assigned functions of the direction keys associated with the opposite directions when flip setting is turned on, a user can operate a key, when the user uses the imaging device 11 in the inverted use state, with the same operating sense as when the imaging device 11 is used in the normal use state. Accordingly, it is possible to improve usability of the imaging device 11.

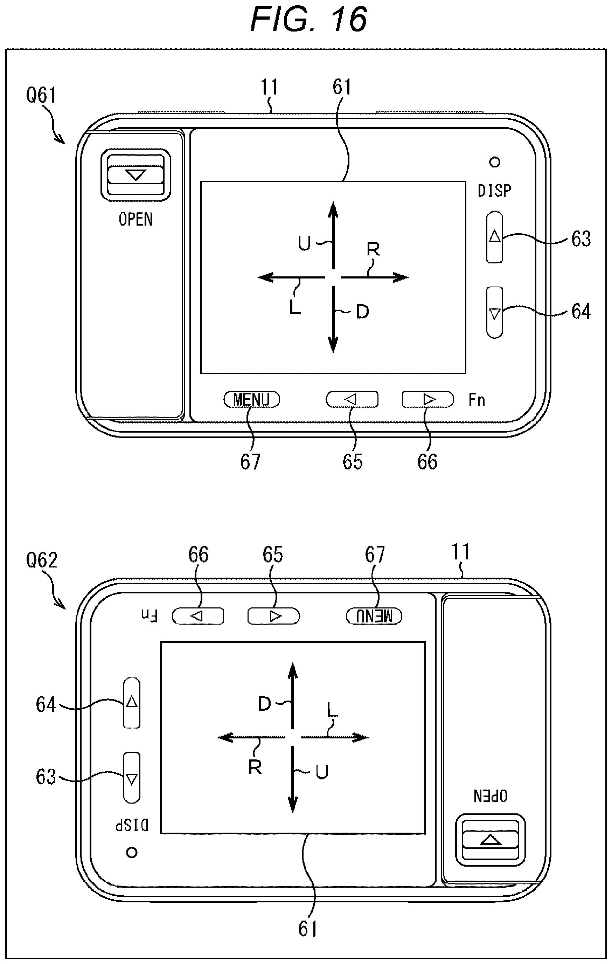

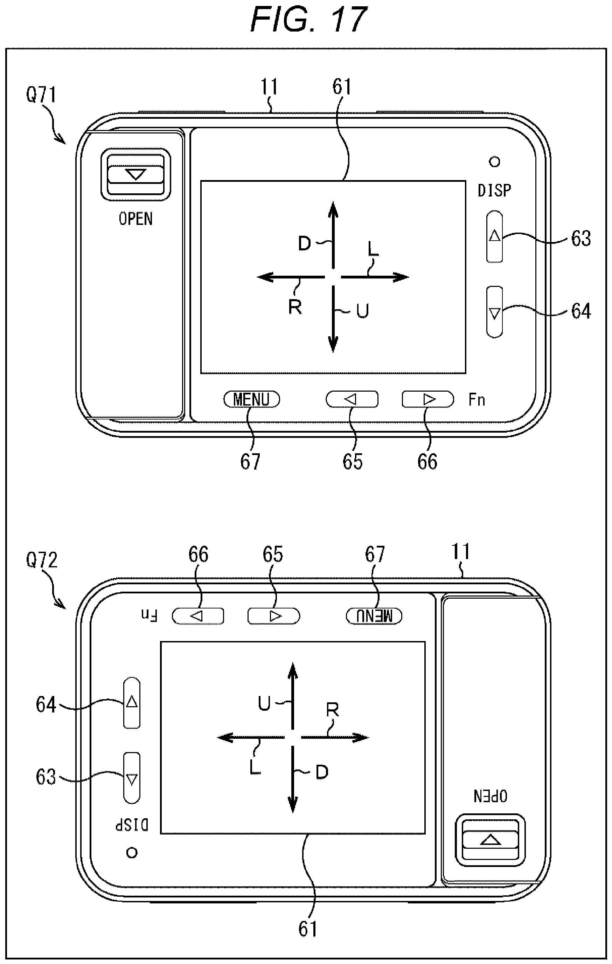

Accordingly, behavior of the imaging device 11 when the direction keys are operated, for example, as indicated by Figs. 16 and 17 varies depending on whether flip setting is turned on or off or the use state of the imaging device 11.

Incidentally, elements in Figs. 16 and 17 corresponding to those in Fig. 3 are referred to by the same reference signs and description thereof will be appropriately skipped. Further, in Figs. 16 and 17, an arrow U, an arrow D, an arrow L, and an arrow R denote the screen upward direction, the screen downward direction, the screen leftward direction, and the screen rightward direction, respectively.

For example, when flip setting is turned off and a user operates the direction keys in the normal use state as indicated by an arrow Q61 in Fig. 16, the operation is performed in the directions of the functions assigned to the direction keys in initial setting and in the same directions as seen from the user. Here, assignment of a function to each direction key in the initial setting refers to assignment of a function in the state in which flip setting is turned off.

When the upward button 63, the downward button 64, the leftward button 65, and the rightward button 66 are operated, the upward, downward, leftward, and rightward operations in Fig. 16 are performed. In addition, in the example indicated by the arrow Q61, the upward, downward, leftward, and rightward directions in the drawing are the screen upward direction, the screen downward direction, the screen leftward direction, and the screen rightward direction, respectively.

Accordingly, for example, when a user operates the upward button 63 in a state in which a cursor is displayed on the display unit 61, the cursor moves in the upward direction which is a direction of an arrow printed in the upward button 63 in the drawing. Further, in the drawing, the upward direction is a direction toward the top end of the screen displayed on the display unit 61, that is, the screen upward direction.

Further, for example, when flip setting is turned off and a user operates the direction keys in the inverted use state as indicated by an arrow Q62 in Fig. 16, the operation is performed in the directions of the functions assigned to the direction keys in initial setting and in the same directions as seen from the user.

That is, when the upward button 63, the downward button 64, the leftward button 65, and the rightward button 66 are operated, the downward, upward, rightward, and leftward operations in Fig. 16 are performed. Here, in the example indicated by the arrow Q62, the upward, downward, leftward, and rightward directions in the drawing are the screen downward direction, the screen upward direction, the screen rightward direction, and the screen leftward direction, respectively.

Accordingly, for example, when a user operates the upward button 63 in a state in which a cursor is displayed on the display unit 61, the cursor moves in the downward direction which is a direction of an arrow printed in the upward button 63 in the drawing. Further, in the example indicated by the arrow Q62, the downward direction in the drawing is a direction toward the top end of the screen displayed on the display unit 61, that is, the screen upward direction.

Further, when flip setting is turned on, the assigned functions of the upward button 63 and the downward button 64 are exchanged and the assigned functions of the leftward button 65 and the rightward button 66 are exchanged.

Accordingly, for example, when flip setting is turned on and a user operates the direction keys in the normal use state as indicated by an arrow Q71 in Fig. 17, assignment of the functions to the direction keys is different from that when flip setting is turned off, but the operation is performed in the same directions as seen from the user.

That is, when the upward button 63, the downward button 64, the leftward button 65, and the rightward button 66 are operated, the upward, downward, leftward, and rightward operations in Fig. 17 are performed. Further, in the example indicated by the arrow Q71, the upward, downward, leftward, and rightward directions in the drawing are the screen downward direction, the screen upward direction, the screen rightward direction, and the screen leftward direction, respectively.

Accordingly, for example, when a user operates the upward button 63 in a state in which a cursor is displayed on the display unit 61, the cursor moves in the upward direction which is a direction of an arrow printed in the upward button 63 in the drawing. Here, in the drawing, the upward direction is a direction toward the bottom end of the screen displayed on the display unit 61, that is, the screen downward direction.

When flip setting is turned on in this way, the function of the operation associated with the downward direction which is opposite to the upward direction, that is, the screen downward direction, with respect to the display screen is assigned to the upward button 63 associated with the upward direction, and the functions are assigned to the other direction keys in the similar way.

Further, for example, when flip setting is turned on and a user operates the direction keys in the inverted use state as indicated by an arrow Q72 in Fig. 17, assignment of the functions to the direction keys is different from that when flip setting is turned off, but the operation is performed in the same directions as seen from the user.

That is, when the upward button 63, the downward button 64, the leftward button 65, and the rightward button 66 are operated, the downward, upward, rightward, and leftward operations in Fig. 17 are performed. Here, in the example indicated by the arrow Q72, the upward, downward, leftward, and rightward directions in the drawing are the screen upward direction, the screen downward direction, the screen leftward direction, and the screen rightward direction, respectively.

Accordingly, for example, when a user operates the upward button 63 in a state in which a cursor is displayed on the display unit 61, the cursor moves in the downward direction which is a direction of an arrow printed in the upward button 63 in the drawing. Further, in the example indicated by the arrow Q72, the downward direction in the drawing is a direction toward the bottom end of the screen displayed on the display unit 61, that is, the screen downward direction.

In the state in which flip setting is turned on in this way, the functions assigned to the direction keys are exchanged.

Accordingly, a direction of an operation which is performed when a user operates a direction key is opposite to the direction associated with the direction key with respect to the screen such as the menu screen or the recording image. That is, when the upward button 63 associated with the upward direction is operated, the operation associated with the downward direction, that is, the screen downward direction, with respect to the display screen is performed, for example.

However, when flip setting is turned on, the display screen is vertically inverted and displayed, that is, the display direction is vertically inverted. Accordingly, when a user operates the direction keys, the operation is performed in the directions of the arrows printed in the direction keys. That is, the operation is performed in the same directions as seen from the user.

Accordingly, a user can perform an operation with the similar operating sense to when flip setting is turned off and it is thus possible to improve usability of the imaging device 11.

Further, in the state in which flip setting is turned on, the functions of the operations associated with no direction are exchanged between the direction keys.

Accordingly, even when a user turns on the flip setting and uses the imaging device 11 in the inverted use state, the user can perform an operation with the same operating sense as when the flip setting is turned off and the imaging device 11 is used in the normal use state and it is thus possible to further improve usability.

Specifically, for example, when the flip setting is turned off as in the example indicated by the arrow Q61 in Fig. 16 and the upward button 63 in which an upward arrow when seen by the user is printed is operated during capturing of a moving image or during reproduction of a recording medium in the normal use state, the DISP function is performed.

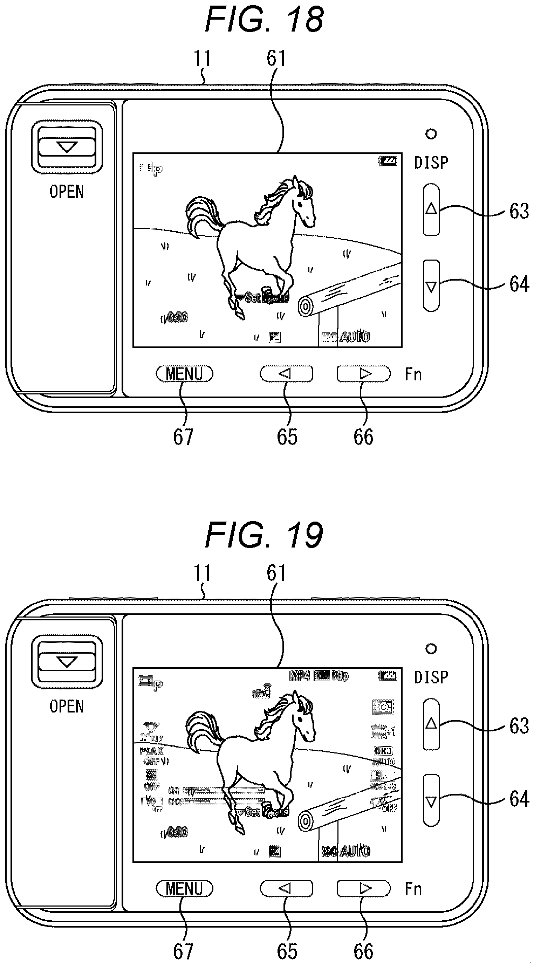

That is, when the upward button 63 to which the DISP function is assigned is operated by the user, display on the display unit 61 of the imaging device 11 is switched as illustrated in Figs. 18 to 20.

For example, in the example illustrated in Fig. 18, an OSD image including minimum necessary information such as an imaging mode or an amount of power remaining in a battery is displayed on the display unit 61.

Further, in the example illustrated in Fig. 19, an OSD image including more information than the OSD image illustrated in Fig. 18, such as a photometrymode or setting of white balance adjustment, is displayed on the display unit 61.

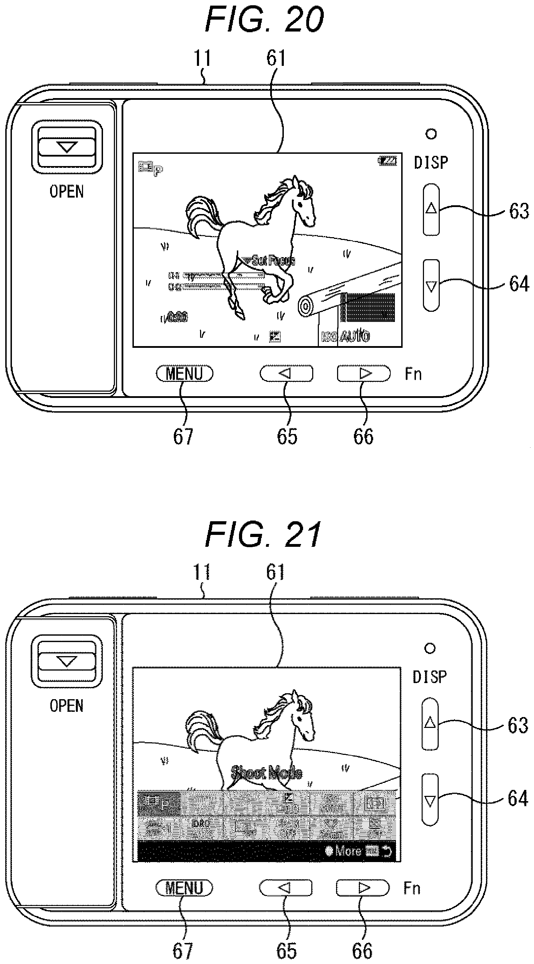

Furthermore, in the example illustrated in Fig. 20, an OSD image including some information including a luminance histogram of a through-image or a recording image in addition to the similar minimum necessary information to the OSD image illustrated in Fig. 18 is displayed on the display unit 61.

Whenever a user operates the upward button 63 to which the DISP function is assigned, the display on the display unit 61 is switched, for example, from the display illustrated in Fig. 18 to the display illustrated in Fig. 19 and is further switched to the display illustrated in Fig. 20. In this way, switching of the display is performed.

At this time, since characters "DISP" are printed in the vicinity of the upward button 63 to which the DISP function is assigned, a user can easily switch the display.

On the other hand, for example, when the flip setting is turned on as in the example indicated by the arrow Q72 in Fig. 17 and the downward button 64 in which an upward arrow when seen by the user is printed is operated during capturing of a moving image or during reproduction of a recording medium in the inverted use state, the DISP function is performed.

In this way, the direction key (the button) to which the DISP function is assigned varies depending on whether the flip setting is turned on or off. However, in any case, when a user who uses the imaging device 11 in the use state supposed for the flip setting operates the direction key in which an upward arrow is printed when seen from the user, the DISP function can be performed. That is, a user can perform the operation for the DISP function with the same operating sense without depending on the flip setting.

Similarly to the DISP function, a user can perform the operation for the reproduction function with the same operating sense without depending on whether the flip setting is turned on or off.

That is, for example, when the flip setting is turned off as in the example indicated by the arrow Q61 in Fig. 16 and the imaging device is used in the normal use state, a user can instruct to perform the reproduction function by operating the downward button 64 in which the downward arrow is printed when seen from the user.

On the other hand, for example, when the flip setting is turned on as in the example indicated by the arrow Q72 in Fig. 17 and the imaging device is used in the inverted use state, a user can instruct to perform the reproduction function by operating the upward button 63 in which the downward arrow is printed when seen from the user.

Further, as for the leftward button 65 and the rightward button 66 as well as the upward button 63 and the downward button 64, a user can perform the operation with the similar operating sense without depending on the flip setting.

That is, for example, when the flip setting is turned off as in the example indicated by the arrow Q61 in Fig. 16 and a user operates the rightward button 66 during capturing of a moving image in the normal use state, the Fn function assigned to the rightward button 66 is performed.

Specifically, when the rightward button 66 is operated, for example, a function setting screen illustrated in Fig. 21 is displayed on the display unit 61. In this example, various settings such as an imaging mode, an exposure, an ISO sensitivity, a photometry mode, and white balance adjustment can be performed on the function setting screen.

On the other hand, for example, when the flip setting is turned on as in the example indicated by the arrow Q72 in Fig. 17 and the imaging device is used in the inverted use state, a user can display the function setting screen illustrated in Fig. 21 on the display unit 61 by operating the leftward button 65. That is, the Fn function can be performed.

In this way, without depending on whether the flip setting is turned on or off, a user can instruct to perform the Fn function by operating the direction key (the button) in which the rightward arrow is printed when seen from the user.

Furthermore, for example, when the flip setting is turned off as in the example indicated by the arrow Q61 in Fig. 16 and a user operates the leftward button 65 during capturing of a moving image or the like in the normal use state, the WB setting function assigned to the leftward button 65 is performed.

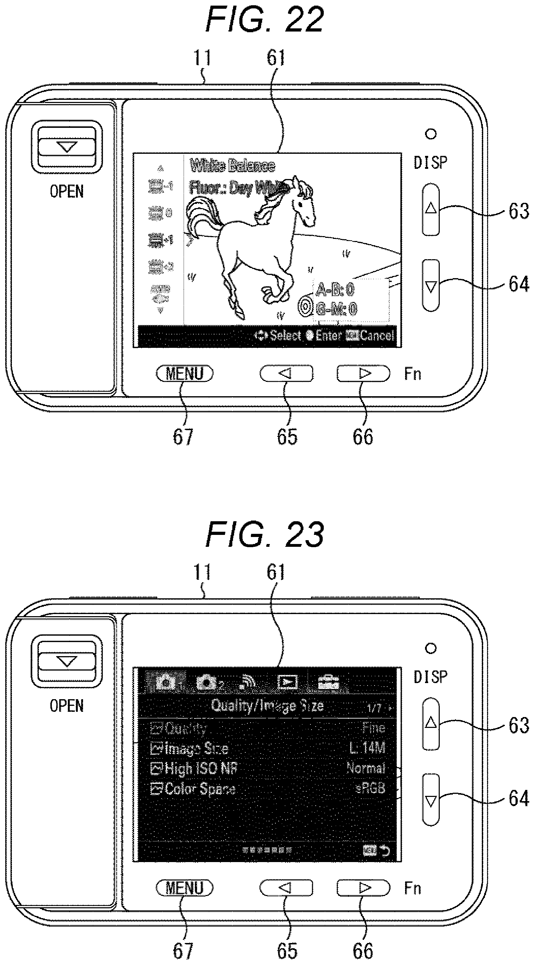

Specifically, when the leftward button 65 is operated, for example, a WB setting screen illustrated in Fig. 22 is displayed on the display unit 61. In this example, an imaging environment in white balance adjustment such as a clear sky, a fluorescent light, and automation can be set on the WB setting screen.

On the other hand, for example, when the flip setting is turned on as in the example indicated by the arrow Q72 in Fig. 17 and the imaging device is used in the inverted use state, a user can display the WB setting screen illustrated in Fig. 22 on the display unit 61 by operating the rightward button 66. That is, the WB setting function can be performed. Here, in this case, since the flip setting is turned on, the WB setting screen is displayed in the inverted display state.

In this way, without depending on whether the flip setting is turned on or off, a user can instruct to perform the WB setting function by operating the direction key (the button) in which the leftward arrow is printed when seen from the user.

Furthermore, as described above, the assigned function of a button associated with no direction such as the menu button 67 is not changed even when the flip setting is turned on.

Accordingly, when the menu button 67 is operated by a user, for example, the menu screen illustrated in Fig. 23 is displayed on the display unit 61 without depending on whether the flip setting is turned on or off.

A user can perform various settings by performing an operation such as selection or determination on the menu screen displayed in this way. For example, in the example illustrated in Fig. 23, a user can perform setting of the quality of a recording image, setting of the size of a recording image, or the like.

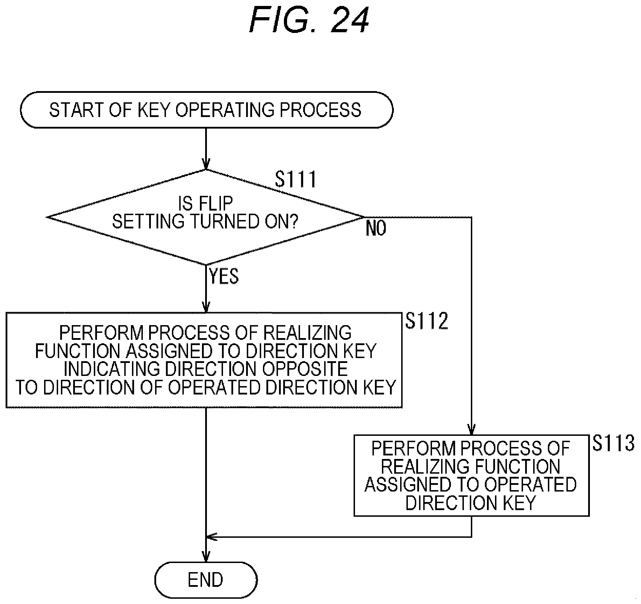

A key operating process of causing the imaging device 11 to perform a process corresponding to an operation when the direction keys are operated as described above will be described below. That is, the key operating process which is performed by the imaging device 11 will be described below with reference to the flowchart illustrated in Fig. 24.

This key operating process is started, for example, when one direction key of the imaging device 11 is operated by a user. When a direction key serving as the operation unit 132 is operated by a user, a signal corresponding to the user's operation is supplied from the operation unit 132 to the control unit 133.

In Step S111, the control unit 133 determines whether or not the flip setting is turned on, on the basis of the flip setting information recorded in the setting unit 151.

When it is determined in Step S111 that the flip setting is turned on, the process flow transitions to Step S112.

In Step S112, the control unit 133 performs a process of realizing the function which is assigned in advance to the direction key associated with a direction opposite to the direction of the operated direction key on the basis of a signal based on a user's operation and supplied from the operation unit 132 and the determination result of Step S111. Here, the function assigned in advance refers to a function which is assigned in an initial set state, that is, in a state in which the flip setting is turned off.

In other words, in Step S112, the control unit 133 exchanges the assigned functions of the direction keys on the basis of the determination result of Step S111. In addition, the control unit 133 performs a process of realizing the function which is assigned after the assigned functions have been exchanged on the direction key which is operated by the user on the basis of a signal supplied from the operation unit 132.

Accordingly, for example, it is assumed that the upward button 63 is operated by the user in a state in which the menu screen is displayed on the display unit 61.

In this case, the control unit 133 identifies the downward button 64 associated with the downward direction which is opposite to the upward direction associated with the upward button 63 which is operated by the user, and performs a process of realizing the downward operating function which is assigned to the downward button 64 in the state in which the flip setting is turned off. That is, the display control unit 153 of the control unit 133 controls the display unit 61 such that the cursor displayed on the menu screen is moved in the screen downward direction.

When the flip setting is turned on, aprocessof a function based on the assignment when the flip setting is turned on can be performed by performing the assigned function which is assigned to the direction key which is opposite to the direction of the operated direction key in the state in which the flip setting is turned off.

When the process based on the operation of the direction key is performed in this way, the key operating process ends.

On the other hand, when it is determined in Step S111 that the flip setting is not turned on, that is, the flip setting is turned off, the process flow transitions to Step S113.

In Step S113, the control unit 133 performs a process of realizing the function which is assigned in advance to the operated direction key on the basis of a signal based on the user's operation and supplied from the operation unit 132 and the determination result of Step S111, and then ends the key operating process.

In this case, the control unit 133 performs the function which is assigned to the operated direction key in the state in which the flip setting is turned off. Accordingly, for example, when the upward button 63 is operated by the user in the state in which the menu screen is displayed on the display unit 61, the display control unit 153 of the control unit 133 controls the display unit 61 such that the cursor displayed on the menu screen is moved in the screen upward direction.

In this way, the imaging device 11 performs the process based on the operation of the direction key on the basis of the flip setting and the function which is assigned in advance to the direction key. That is, the imaging device 11 exchanges the functions of the direction keys depending on the flip setting and performs the processes based on the operations of the direction keys. Accordingly, a user can be caused to perform an operation with the same operating sense without depending on the flip setting and thus it is possible to improve usability of the imaging device 11.

Incidentally, an example in which the functions assigned to the direction keys associated with the opposite directions are exchanged has been described above. This is because it is assumed that the imaging device 11 is used in the inverted use state.

However, for example, it is conceivable that the imaging device 11 is used in a state in which it is rotated 90 degrees in the clockwise direction or the counterclockwise direction with an optical axis of the imaging device 11 as a rotation axis. Setting based on such assumption of use may be performed through the flip setting.

In this case, for example, when it is assumed that the imaging device 11 is used in the state in which it is rotated 90 degrees, for example, a function which is assigned in advance to the rightward button 66 may be assigned to the upward button 63 and a function which is assigned in advance to the downward button 64 may be assigned to the rightward button 66. That is, a function which is assigned in advance to a direction key associated with a predetermined direction may be assigned to a direction key associated with a direction forming a predetermined angle such as 90 degrees with respect to the predetermined direction. By exchanging the assigned functions of four direction keys in this way, a user can be caused to perform an operation with the similar operating sense to in the normal use state even when the imaging device 11 is used in the state in which it is rotated 90 degrees.

### <Operation in Remote Operation Unit>

On the other hand, when a user remotely operates the imaging device 11 with the remote operation unit 121 which is an external device, the user operates the remote operation unit 121 while seeing the menu screen or the like displayed on the display unit 61.

At this time, a command based on the user's operation of the remote operation unit 121 is transmitted from the remote operation unit 121 to the imaging device 11, and the imaging device 11 executes the command received from the remote operation unit 121 without depending on whether the flip setting is turned on or off.

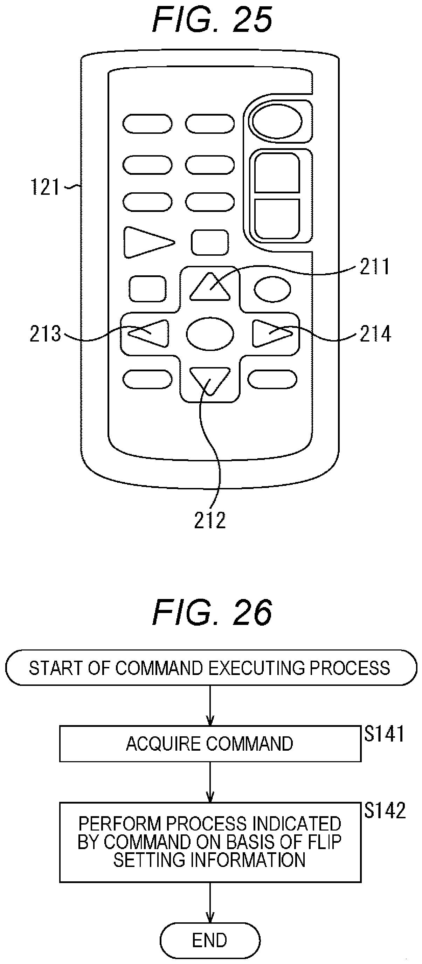

For example, it is assumed that an upward button 211, a downward button 212, a leftward button 213, and a rightward button 214 are provided in the remote operation unit 121 as illustrated in Fig. 25. Incidentally, Fig. 25 illustrates an example of an appearance configuration of the remote operation unit 121.

The upward button 211 is a button to which the upward operating function and the DISP function are assigned and corresponds to the upward button 63 of the imaging device 11.

Further, the downward button 212 is a button to which the downward operating function and another selected function are assigned and corresponds to the downward button 64 of the imaging device 11. Here, it is assumed that the reproduction function is assigned as another selected function to the downward button 212.

The leftward button 213 is a button to which the leftward operating function and another selected function are assigned and corresponds to the leftward button 65 of the imaging device 11. Here, it is assumed that the WB setting function is assigned as another selected function to the leftward button 213.

The rightward button 214 is a button to which the rightward operating function and the Fn function are assigned and corresponds to the right ward button 66 of the imaging device 11.

The upward button 211, the downward button 212, the leftward button 213, and the rightward button 214 are buttons (direction keys) associated with the upward, downward, leftward, and rightward directions, respectively, similarly to the direction keys of the imaging device 11.

Particularly, in the remote operation unit 121, since the upward button 211, the downward button 212, the leftward button 213, and the rightward button 214 are disposed on the upper, lower, left, and right sides when seen from the user, the user can intuitively understand what directions the buttons (the direction keys) are associated with on the basis of the disposed positions thereof.

For example, it is assumed that the flip setting is turned off as indicated by the arrow Q61 in Fig. 16 and a user operates one direction key of the remote operation unit 121 in the state in which the imaging device 11 is used in the normal use state. In this case, an operation is performed in the direction of the function assigned to the direction key and in the same direction as seen from the user.

That is, when the upward button 211, the downward button 212, the leftward button 213, and the rightward button 214 are operated, operations in the upward, downward, leftward, and rightward directions in Fig. 16 are performed. In the example indicated by the arrow Q61, the upward, downward, leftward, and rightward directions in the drawing are the screen upward direction, the screen downward direction, the screen leftward direction, and the screen rightward direction, respectively.

Further, for example, when the flip setting is turned off as indicated by the arrow Q62 in Fig. 16 and a user operates one direction key of the remote operation unit 121 in the state in which the imaging device 11 is used in the inverted use state, an operation is performed in the direction of the function assigned to the direction key but in the direction opposite to the direction seen from the user.

That is, when the upward button 211, the downward button 212, the leftward button 213, and the rightward button 214 are operated, the operations in the downward, upward, rightward, and leftward directions in Fig. 16 are performed. In this way, in the example indicated by the arrow Q62, a user's operation of a direction key is seen as if an operation is performed in a direction opposite to the direction seen from the user.

However, in the example indicated by the arrow Q62, since the upward, downward, leftward, and rightward directions in the drawing are the screen downward direction, the screen upward direction, the screen rightward direction, and the screen leftward direction, respectively, processing is actually performed as the remote operation unit 121 is operated.

Similarly, for example, when the flip setting is turned on as indicated by the arrow Q71 in Fig. 17 and a user operates one direction key of the remote operation unit 121 in the state in which the imaging device 11 is used in the normal use state, an operation is performed in the direction of the function assigned to the direction key but in the direction opposite to the direction seen from the user.

That is, when the upward button 211, the downward button 212, the leftward button 213, and the rightward button 214 are operated, the operations in the downward, upward, rightward, and leftward directions in Fig. 17 are performed.

On the other hand, for example, when the flip setting is turned on as indicated by the arrow Q72 in Fig. 17 and a user operates one direction key of the remote operation unit 121 in the state in which the imaging device 11 is used in the inverted use state, an operation is performed in the direction of the function assigned to the direction key and in the same direction as seen from the user.

That is, when the upward button 211, the downward button 212, the leftward button 213, and the rightward button 214 are operated, operations in the upward, downward, leftward, and rightward directions in Fig. 17 are performed. In the example indicated by the arrow Q72, since the upward, downward, leftward, and rightward directions in the drawing are the screen upward direction, the screen downward direction, the screen leftward direction, and the screen rightward direction, respectively, processing is actually performed in the same directions as seen from the user as the remote operation unit 121 is operated.

Incidentally, the operations associated with the in-plane direction of the display screen have been described herein, and other functions assigned to the direction keys of the remote operation unit 121 are not exchanged. Accordingly, for example, when a user operates the upward button 211 during capturing of an image or during reproduction of an image, the process of realizing the DISP function is performed without depending on whether the flip setting in the imaging device 11 is turned on or off.

By prohibiting exchange of a function such as inversion of a direction for an operation on the remote operation unit 121 unlike the direction keys of the main body of the imaging device 11 as described above, a user can be caused to perform an operation with the same operating sense without depending on the flip setting.

That is, when a user who uses the imaging device 11 in a use state in which the flip setting is assumed operates a direction key of the remote operation unit 121 without depending on whether the flip setting is turned on or off, an operation in the direction of the direction key is performed in the imaging device 11. Accordingly, it is possible to perform an operation without giving discomfort to a user and to improve usability.

For example, regarding the imaging device 11, the imaging device 11 is assumed to be used in the inverted use state when the flip setting is turned on, that is, the imaging device 11 is assumed to be used in the vertically inverted state. Accordingly, when the assigned functions of the direction keys associated with opposite directions are exchanged therebetween, the usability thereof is more excellent.

On the other hand, regarding the remote operation unit 121, no user uses the remote operation unit 121 in the vertically inverted state without depending on whether the flip setting is turned on or off. Accordingly, when the functions assigned to the direction keys of the remote operation unit 121 are not exchanged, the usability thereof is more excellent.

Furthermore, in this case, since the upward, downward, leftward, and rightward directions on a display screen of the menu screen, a recording image, or the like can be identified with reference to the flip setting information in the imaging device 11, a command from the remote operation unit 121 can be rightly executed on the basis of the identification result.

By executing a command with reference to the flip setting information on the imaging device 11 side in this way, an operation can be performed on the remote operation unit 121 side without depending on whether the flip setting is turned on or off.

Incidentally, the behavior of the imaging device 11 when the direction keys of the remote operation unit 121 are operated has been described herein, but even when the imaging device 11 is remotely operated using a remote operation unit 123 for an external device, the behavior of the imaging device 11 is similar to when the remote operation unit 121 is operated.

In this case, for example, a menu screen or a recording image which is supplied from the display control unit 153 of the imaging device 11 via the input and output terminal 136 is displayed on the display unit 181 of the external device 122. Then, a user operates the direction keys or the like of the remote operation unit 123 for an external device while seeing the display unit 181 on which the menu screen or the like is displayed.

Then, a command based on the user's operation is transmitted from the remote operation unit 123 for an external device, and this command is received by the external device 122. The external device 122 supplies the command received from the remote operation unit 123 for an external device in this way to the control unit 133 via the input and output terminal 136, and the control unit 133 supplied with the command executes the command.

When the command is executed in this way, the menu screen or the like which is supplied from the display control unit 153 to the display unit 181 of the external device 122 via the input and output terminal 136 includes the execution result of the command.

A command executing process of causing the imaging device 11 to execute a command received from the remote operation unit 121 or the remote operation unit 123 for an external device as described above will be described below. That is, the command executing process which is performed by the imaging device 11 will be described below with reference to the flowchart illustrated in Fig. 26. Incidentally, when the command executing process is performed, the display control unit 153 of the control unit 133 in the imaging device 11 performs control such that a display screen is displayed in the normal display state or the inverted display state on the basis of the flip setting information.

In Step S141, the control unit 133 acquires a command output from the remote operation unit 121 which is an external device or the remote operation unit 123 for an external device.

For example, the control unit 133 acquires the command, which is transmitted from the remote operation unit 121 and is received by the communication unit 135, from the communication unit 135. Further, for example, the control unit 133 acquires a command, which is transmitted from the remote operation unit 123 for an external device, from the external device 122 via the input and output terminal 136.

In Step S142, the control unit 133 performs a process which is indicated by the acquired command on the basis of the flip setting information recorded in the setting unit 151 and ends the command executing process. That is, the control unit 133 executes the command which is output from the remote operation unit 121 or the remote operation unit 123 for an external device on the basis of the setting of the flip function.

For example, when a command for instructing to operate the upward button 211 or the like in a predetermined direction with respect to the remote operation unit 121 is received, the control unit 133 performs control such that an operation associated with the predetermined direction is performed with respect to the display screen. Specifically, for example, it is assumed that a user operates the upward button 211 of the remote operation unit 121 to instruct an operation associated with the upward direction with respect to the remote operation unit 121, a command based on the operation is acquired, and information indicating that the flip setting is turned on is recorded as the flip setting information in the setting unit 151.

In this case, for example, when the menu screen is displayed in the inverted display state on the display unit 61, the display control unit 153 of the control unit 133 performs a process of moving a cursor on the menu screen in the screen upward direction with respect to the menu screen and selecting a menu item using the cursor as a process indicated by the command. At this time, since the display control unit 153 can identify that the direction toward the bottom surface of the imaging device 11 is the screen upward direction by referring to the flip setting information, it is possible to move the cursor in the right direction indicated by the command.

Further, for example, it is assumed that a user operates the upward button 211 of the remote operation unit 121, a command based on the operation is acquired, and information indicating that the flip setting is turned off is recorded as the flip setting information in the setting unit 151.

In this case, for example, when the menu screen is displayed in the normal display state on the display unit 61, the display control unit 153 of the control unit 133 performs a process of moving a cursor on the menu screen in the screen upward direction with respect to the menu screen and selecting a menu item using the cursor as a process indicated by the command.

Furthermore, for example, when a command instructing an operation in a predetermined direction is received in the state in which an image under reproduction or the like is displayed on the display unit 61 in addition to the state in which the menu screen is displayed, control is performed such that an operation associated with the predetermined direction such as a scrolling operation is performed with respect to the display screen.

Furthermore, for example, it is assumed that a user operates the upward button 211 of the remote operation unit 121 and a command based on the operation is acquired. In this case, for example, when a moving image is being captured or a recording image is being reproduced, the display control unit 153 of the control unit 133 controls the display unit 61 such that a process of realizing the DISP function is performed as a process indicated by the command. Specifically, for example, a process of switching the display which has been described above with reference to Figs. 18 to 20 is performed as the process of realizing the DISP function.

In this way, for example, when a command instructing an operation associated with a predetermined direction, that is, an operation in the predetermined direction, such as the upward operating function is acquired, the control unit 133 identifies whether the display screen is displayed in the vertically inverted or the like with reference to the flip setting information. Then, the control unit 133 executes the acquired command such that the operation associated with the predetermined direction is performed with respect to the display screen, that is, such that the operation in the predetermined direction is performed with respect to the display screen.

On the other hand, for example, when a command instructing an operation associated with no direction such as the DISP function is acquired, the control unit 133 executes the command without any change such that the operation associated with no direction is performed without depending on when the flip setting is turned on or off. That is, control is performed such that the operation associated with no direction is performed.

As described above, the imaging device 11 acquires a command output from the remote operation unit 121 or the remote operation unit 123 for an external device and executes the command on the basis of the flip setting information.

By executing the command on the basis of the flip setting information in this way, the remote operation unit 121 or the remote operation unit 123 for an external device can perform an operation without depending on whether the flip setting is turned on or off and it is thus possible to improve the usability.

### <Example of Configuration of Computer>

Incidentally, the above-mentioned series of processes can be performed in hardware or can be performed in software. When the series of processes are performed in software, a program constituting the software is installed in a computer. Here, examples of the computer include a computer which is incorporated into dedicated hardware, a general-purpose personal computer that can perform various functions by installing various programs therein, and the like.

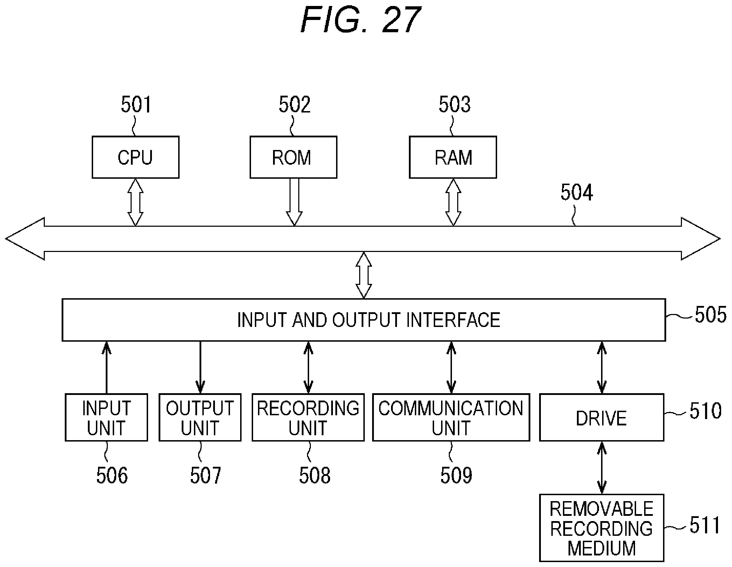

Fig. 27 is a block diagram illustrating an example of a hardware configuration of a computer that performs the above-mentioned series of processes in accordance with a program.

In the computer, a central processing unit (CPU) 501, a read only memory (ROM) 502, and a random access memory (RAM) 503 are connected to each other via a bus 504.

An input and output interface 505 is further connected to the bus 504. An input unit 506, an output unit 507, a recording unit 508, a communication unit 509, and a drive 510 are connected to the input and output interface 505.

The input unit 506 is constituted by an input switch, a button, a microphone, an imaging element, or the like. The output unit 507 is constituted by a display, a speaker, or the like. The recording unit 508 is constituted by a hard disk, a nonvolatile memory, or the like. The communication unit 509 is constituted by a network interface and the like. The drive 510 drives a removable recording medium 511 such as a magnetic disk, an optical disc, a magneto-optical disc, or a semiconductor memory.

In the computer having the above-mentioned configuration, the above-mentioned series of processes are performed, for example, by causing the CPU 501 to load a program recorded in the recording unit 508 into the RAM 503 via the input and output interface 505 and the bus 504 and to execute the loaded program.

The program which is executed by the computer (the CPU 501) can be recorded, for example, on the removable recording medium 511 serving as a package medium or the like and can be provided. Further, the program can be provided via a wired or wireless transmission medium such as a local area network, the Internet, and a digital satellite broadcast.

In the computer, a program can be installed in the recording unit 508 via the input and output interface 505 by setting the removable recording medium 511 to the drive 510. Further, a program can be received by the communication unit 509 via a wired or wireless transmission medium and can be installed in the recording unit 508. In addition, a program can be installed in advance in the ROM 502 or the recording unit 508.

Incidentally, a program which is executed by the computer may be a program for performing processes in a time series in accordance with a sequence described in this specification or may be a program for performing processes in parallel or at a necessary timing such as when it is called.

Further, an embodiment of the present technology is not limited to the above-mentioned embodiment and can be modified in various forms without departing from the gist of the present technology.

For example, the present technology may have a configuration of cloud computing in which one function is distributed to a plurality of devices via a network and is processed in common.

Further, the steps described in the above-mentioned flowcharts may be performed by one device or may be distributed and performed by a plurality of devices.

Furthermore, when one step includes a plurality of processes, the plurality of processes included in one step may be performed by one device or may be distributed and performed by a plurality of devices.

### <Application Example>

The technology according to the present disclosure can be applied to various products. For example, the technology according to the present disclosure may be applied to an endoscopic surgery system.

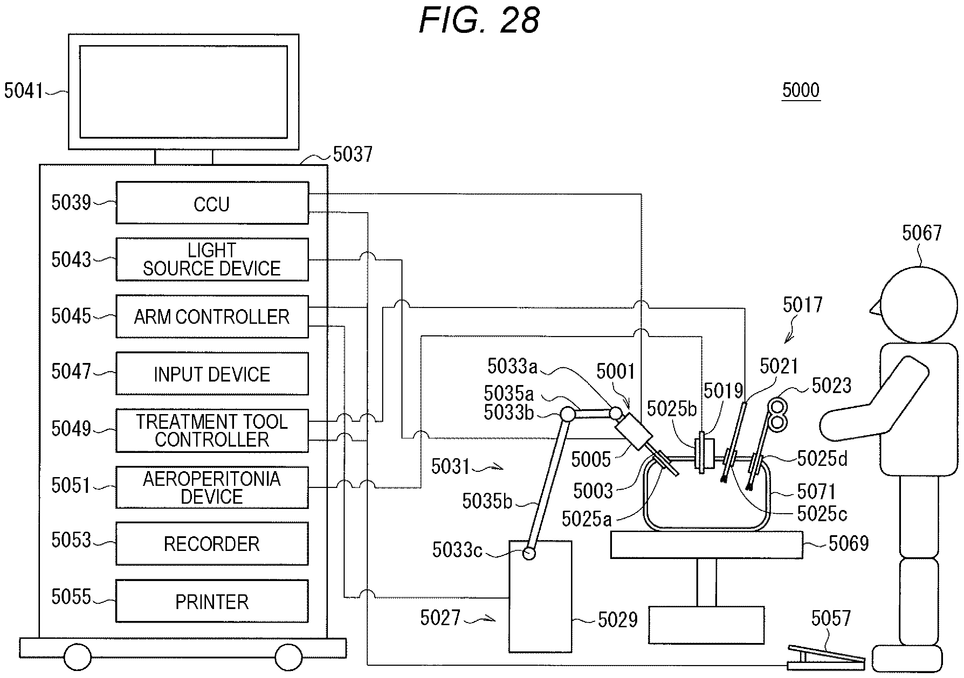

Fig. 28 is a diagram illustrating an example of a schematic configuration of an endoscopic surgery system 5000 to which the technology according to the present disclosure can be applied. In Fig. 28, a state in which an operator (a doctor) 5067 performs a surgical operation on a patient 5071 on a patient bed 5069 using the endoscopic surgery system 5000 is illustrated. As illustrated in the drawing, the endoscopic surgery system 5000 includes an endoscope 5001, other surgical instruments 5017, a support arm unit 5027 that supports the endoscope 5001, and a cart 5037 on which various devices for an endoscopic surgical operation are mounted.

In an endoscopic surgical operation, a plurality of tubular puncturing tools, which are called trocars 5025a to 5025d, puncture an abdominal wall instead of cutting and opening the abdominal wall. Then, a lens barrel 5003 of the endoscope 5001 or the other surgical instruments 5017 are inserted into a body cavity of the patient 5071 from the trocars 5025a to 5025d. In the illustrated example, an aeroperitonia tube 5019, an energy treatment tool 5021, and a forceps 5023 are inserted into the body cavity of the patient 5071 as the other surgical instruments 5017. Further, the energy treatment tool 5021 is a treatment tool that is used to perform cutting and separation of a tissue, sealing of a blood vessel, or the like using a high-frequency current or ultrasonic vibration. Here, the surgical instruments 5017 illustrated in the drawing are merely examples and, for example, various surgical instruments which are generally used in an endoscopic surgical operation such as tweezers and a retractor may be used as the surgical instruments 5017.

An image of a site of operation in the body cavity of the patient 5071 which is captured using the endoscope 5001 is displayed on a display device 5041. The operator 5067 performs treatment such as cutting an affected area using the energy treatment tool 5021 or the forceps 5023 while seeing the image of the site of operation displayed on the display device 5041 in real time, for example. Incidentally, although not illustrated, the aeroperitonia tube 5019, the energy treatment tool 5021, and the forceps 5023 are supported by the operator 5067, an assistant, or the like during the surgical operation.

### (Support Arm Unit)

The support arm unit 5027 includes an arm portion 5031 that extends from a base portion 5029. In the illustrated example, the arm portion 5031 includes joints 5033a, 5033b, and 5033c and links 5035a and 5035b, and is driven under the control of an arm controller 5045. The endoscope 5001 is supported by the arm portion 5031 and the position and the posture thereof are controlled. Accordingly, stable fixing of the position of the endoscope 5001 can be realized.

### (Endoscope)

The endoscope 5001 includes the lens barrel 5003 of which an area with a predetermined length from the tip thereof is inserted into the body cavity of the patient 5071 and a camera head 5005 that is connected to the base of the lens barrel 5003. In the illustrated example, the endoscope 5001 is illustrated as a so-called rigid endoscope including a rigid lens barrel 5003, but the endoscope 5001 may include a so-called flexible endoscope including a flexible lens barrel 5003.

An opening to which an objective lens is fitted is provided at the tip of the lens barrel 5003. A light source unit 5043 is connected to the endoscope 5001, and light generated by the light source unit 5043 is guided to the tip of the lens barrel by a light guide extending in the lens barrel 5003 and is applied to an observation target in the body cavity of the patient 5071 via the objective lens. Incidentally, the endoscope 5001 may be a direct-view endoscope, an oblique-view endoscope, or a side-view endoscope.

An optical system and an imaging element are provided in the camera head 5005, and reflected light (observation light) from the observation target is focused on the imaging element by the optical system. The observation light is photoelectrically converted by the imaging element and an electrical signal corresponding to the observation light, that is, an image signal corresponding to an observation image, is generated. The image signal is transmitted as RAW data to a camera control unit (CCU) 5039. Incidentally, a function of adjusting a magnification and a focal distance by appropriately driving the optical system is mounted in the camera head 5005.

Incidentally, in order to cope with, for example, stereoscopic vision (3D display) or the like, a plurality of imaging elements may be provided in the camera head 5005. In this case, a plurality of relay optical systems are provided in the lens barrel 5003 in order to guide observation light to the plurality of imaging elements.

### (Various Devices Mounted in Cart)

The CCU 5039 is constituted by a central processing unit (CPU), a graphics processing unit (GPU), or the like and comprehensively controls the operations of the endoscope 5001 and the display device 5041. Specifically, the CCU 5039 performs various image processing for displaying an image based on an image signal received from the camera head 5005, such as a developing process (demosaic processing) or the like, on the image signal. The CCU 5039 provides the image signal having been subjected to the image processing to the display device 5041. Further, the CCU 5039 transmits a control signal to the camera head 5005 and controls driving thereof. The control signal may include information regarding imaging conditions such as a magnification or a focal distance.

The display device 5041 displays an image based on the image signal having been subjected to the image processing by the CCU 5039 under the control of the CCU 5039. When the endoscope 5001 copes with imaging with a high resolution such as 4K (horizontal pixels 3840 × vertical pixels 2160), 8K (horizontal pixels 7680 × vertical pixels 4320), or the like, and/or when the endoscope 5001 copes with 3D display, a display device that can display an image with a high resolution and/or a display device that can display a 3D image can be used as the display device 5041. When the endoscope 5001 copes with imaging with a high resolution such as 4K or 8K, a display device with a size of 55 inches or larger can be used as the display device 5041, whereby it is possible to further obtain a sense of immersion. Further, a plurality of display devices 5041 with different resolutions and sizes may be provided depending on usage.

The light source unit 5043 is constituted, for example, by a light source such as a light emitting diode (LED) and supplies irradiation light that is used to image a site of operation to the endoscope 5001.

The arm controller 5045 is constituted, for example, by a processor such as a CPU and operates in accordance with a predetermined program such that driving of the arm portion 5031 of the support arm unit 5027 is controlled in accordance with a predetermined control system.

The input device 5047 is an input interface for the endoscopic surgery system 5000. A user can perform input of a variety of information or input of an instruction to the endoscopic surgery system 5000 via the input device 5047. For example, a user inputs a variety of information regarding a surgical operation such as body information of a patient or information regarding a surgical operation method via the input device 5047. Further, for example, a user inputs an instruction to drive the arm portion 5031, an instruction to change imaging conditions (such as a type of irradiation light, a magnification, and a focal distance) using the endoscope 5001, an instruction to drive the energy treatment tool 5021, and the like via the input device 5047.

The type of the input device 5047 is not limited and the input device 5047 may include various known input devices. For example, a mouse, a keyboard, a touch panel, a switch, a foot switch 5057, and/or a lever, and the like can be used as the input device 5047. When a touch panel is used as the input device 5047, the touch panel may be provided on a display surface of the display device 5041.

Alternatively, the input device 5047 is a device that is worn by a user such as an eyeglass wearable device, a head mounted display (HMD), or the like, and various inputs are performed on the basis of a user's gesture or sight line which is detected by the device. Further, the input device 5047 includes a camera that can detect a user's movement, and various inputs are performed on the basis of a user's gesture or sight line detected from an image captured by the camera. Moreover, the input device 5047 includes a microphone that can collect a user's sound and various inputs are performed on the basis of sound via the microphone. In this way, by constituting the input device 5047 such that a variety of information is input in a noncontact manner, particularly a user (for example, the operator 5067) in a clean area can operate a device in an unclean area in a noncontact manner. Further, since a user can operate a device without detaching a hand from a carried surgical instrument, it is possible to improve convenience for a user.

A treatment tool controller 5049 controls driving of the energy treatment tool 5021 for cautery or cutting of a tissue, sealing of a blood vessel, or the like. An aeroperitonia device 5051 supplies gas to the body cavity via the aeroperitonia tube 5019 to expand the body cavity of the patient 5071 for the purpose of securement of a view and securement of a work space of an operator using the endoscope 5001. A recorder 5053 is a device that can record a variety of information regarding a surgical operation. A printer 5055 is a device that can print a variety of information regarding a surgical operation in various forms such as text, image, or graph.

Characteristic configurations of the endoscopic surgery system 5000 will be described below in more details.

### (Support Arm Unit)

The support arm unit 5027 includes a base portion 5029 serving as a mount and an arm portion 5031 extending from the base portion 5029. In the illustrated example, thearmportion 5031 includes the plurality of joints 5033a, 5033b, and 5033c and the plurality of links 5035a and 5035b connected to the joint 5033b, but the configuration of the arm portion 5031 is simplified and illustrated in Fig. 28 for the purpose of simplification. Actually, the shapes, the numbers, and the arrangements of the joints 5033a to 5033c and the links 5035a and 5035b, the direction of rotation axes of the joints 5033a to 5033c, and the like can be appropriately set such that the armportion 5031 has a desired degree of freedom. For example, the arm portion 5031 can be suitably configured to have six degrees of freedom or more. Accordingly, since the endoscope 5001 can be freely moved in a movable range of the arm portion 5031, the lens barrel 5003 of the endoscope 5001 can be inserted into the body cavity of the patient 5071 in a desired direction.

Actuators are provided in the joints 5033a to 5033c, and the joints 5033a to 5033c are configured to rotate around predetermined rotation axes by driving the actuators. By controlling the driving of the actuators using the arm controller 5045, the rotation angles of the joints 5033a to 5033c are controlled and driving of the arm portion 5031 is controlled. Accordingly, control of the position and posture of the endoscope 5001 can be realized. At this time, the arm controller 5045 can control driving of the arm portion 5031 using various known control systems such as force control or position control.

For example, by causing the operator 5067 to appropriately perform operation inputs via the input device 5047 (which includes the foot switch 5057), driving of the arm portion 5031 may be appropriately controlled by the arm controller 5045 and the position and posture of the endoscope 5001 may be controlled on the basis of the operation inputs. Through this control, the endoscope 5001 at the tip of the arm portion 5031 can be moved from an arbitrary position to an arbitrary position and can be fixedly supported at the moved position. Incidentally, the arm portion 5031 may be operated in a so-called master-slave system. In this case, the arm portion 5031 can be remotely operated by a user via the input device 5047 which is installed in a place distant from an operation room.

Further, when force control is used, the arm controller 5045 may perform so-called power assist control for receiving an external force from a user and driving the actuators of the joints 5033a to 5033c such that the arm portion 5031 moves smoothly along the external force. Accordingly, a user can move the arm portion 5031 with a relative small force when the user directly touches the arm portion 5031 and moves the arm portion 5031. Accordingly, it is possible to more intuitively move the endoscope 5001 with a simpler operation and to improve convenience for a user.

Here, in general, in an endoscopic surgical operation, the endoscope 5001 is supported by a doctor who is called scopist. On the other hand, since the position of the endoscope 5001 can be more reliably fixed without using a human hand by using the support arm unit 5027, it is possible to stably obtain an image of a site of operation and to smoothly perform a surgical operation.

Incidentally, the arm controller 5045 may not be necessarily provided in the cart 5037. Further, the arm controller 5045 may not be necessarily constituted by a single device. For example, the arm controller 5045 may be provided in each of the joints 5033a to 5033c of the arm portion 5031 of the support arm unit 5027, or driving control of the arm portion 5031 may be realized in cooperation of a plurality of arm controllers 5045.

### (Light Source Unit)

The light source unit 5043 supplies irradiation light for imaging a site of operation to the endoscope 5001. The light source unit 5043 is constituted, for example, by an LED, a laser beam source, or a white light source which is a combination thereof. At this time, when a white light source is constituted in combination of RGB laser beam sources, an output intensity and an output timing of each color (each wavelength) can be controlled with high accuracy and thus white balance adjustment of a captured image in the light source unit 5043 can be performed. Further, in this case, by irradiating an observation target with laser beams from the RGB laser light sources in a time division manner and controlling driving of the imaging element of the camera head 5005 in synchronization with the irradiation timing, images corresponding to RGB colors can also be captured in a time division manner. According to this method, it is possible to obtain a color image without providing a color filter in the imaging element.

Further, driving of the light source unit 5043 may be controlled such that the intensity of output light thereof changes every predetermined time. By controlling driving of the imaging element of the camera head 5005 in synchronization with the change timing of the light intensity such that images are acquired in a time division manner and combining the acquired images, it is possible to generate an image with a high dynamic range without black defects and halation.

Further, the light source unit 5043 may be configured to supply light of a predetermined wavelength band corresponding to special light imaging. In the special light imaging, so-called narrow-band imaging of imaging a predetermined tissue such as a blood vessel of a mucous membrane surface layer is performed, for example, by applying light of a narrower band than that of irradiation light (that is, white light) at the time of normal observation using wavelength dependency of light absorption in a body tissue. Alternatively, in the special light imaging, fluorescent imaging of acquiring an image using fluorescent light which is generated by irradiation with excitation light may be performed. In the fluorescent imaging, irradiating a body tissue with excitation light and observing fluorescent light from the body tissue (auto-fluorescence imaging), or locally injecting a reagent such as indocyanine green (ICG) into a body tissue and irradiating the body tissue with excitation light corresponding to a fluorescence wavelength of the reagent to acquire a fluorescent image, or the like can be performed. The light source unit 5043 can be configured to supply narrow-band light and/or excitation light corresponding to the special light imaging.

### (Camera Head and CCU)

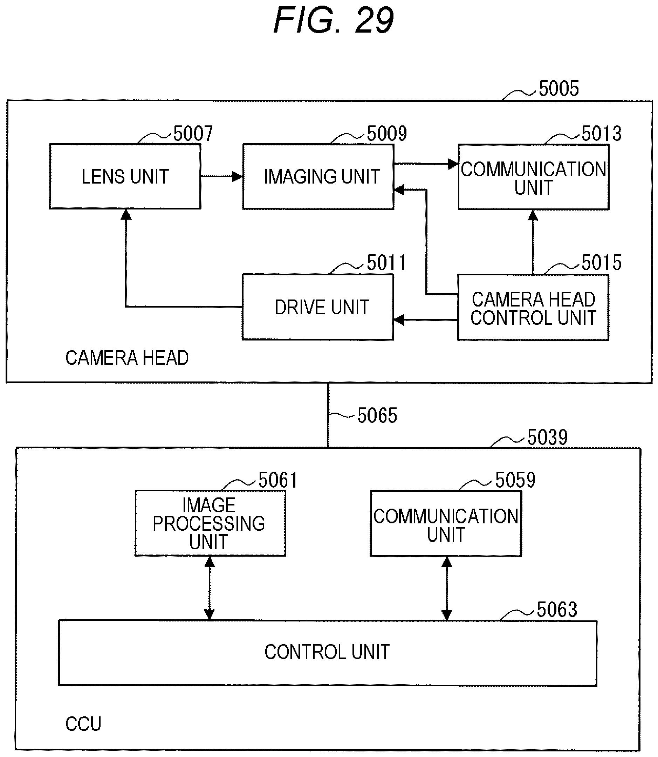

The functions of the camera head 5005 and the CCU 5039 of the endoscope 5001 will be described below in more details with reference to Fig. 29. Fig. 29 is a block diagram illustrating an example of the functional configuration of the camera head 5005 and the CCU 5039 illustrated in Fig. 28.

Referring to Fig. 29, the camera head 5005 includes a lens unit 5007, an imaging unit 5009, a drive unit 5011, a communication unit 5013, and a camera head control unit 5015 as functional units thereof. Further, the CCU 5039 includes a communication unit 5059, an image processing unit 5061, and a control unit 5063 as functional units thereof. The camera head 5005 and the CCU 5039 are connected to each other to perform bidirectional communication therebetween via a transmission cable 5065.

First, the functional configuration of the camera head 5005 will be described. The lens unit 5007 is an optical system that is provided in a connection portion to the lens barrel 5003. Observation light supplied from the tip of the lens barrel 5003 is guided to the camera head 5005 and is incident on the lens unit 5007. The lens unit 5007 is constituted by combination of a plurality of lenses including a zoom lens and a focus lens. Optical characteristics of the lens unit 5007 are adjusted such that observation light is focused on a light receiving surface of an imaging element of the imaging unit 5009. Further, the zoom lens and the focus lens are constituted such that the positions on the optical axis thereof are movable to adjust the magnification and the focal point of a captured image.

The imaging unit 5009 is constituted by an imaging element and is disposed in the rear stage of the lens unit 5007. Observation light passing through the lens unit 5007 is focused on the light receiving surface of the imaging element and an image signal corresponding to an observation image is generated by photoelectric conversion. The image signal generated by the imaging unit 5009 is supplied to the communication unit 5013.

For example, a complementary metal oxide semiconductor (CMOS) type image sensor with a Bayer array that can capture a color image is used as the imaging element constituting the imaging unit 5009. Incidentally, for example, an imaging element that can cope with imaging with a high resolution of 4K or higher may be used as the imaging element. When an image of a site of operation with a high resolution is acquired, the operator 5067 can understand the state of the site of operation in more details and can more smoothly perform a surgical operation.

Further, the imaging element constituting the imaging unit 5009 is configured to have a pair of imaging elements for acquiring each of right-eye and left-eye image signals corresponding to 3D display. By performing 3D display, the operator 5067 can more accurately understand a depth of a biological tissue in a site of operation. Incidentally, when the imaging unit 5009 is constituted in a multi-plate system, a plurality of lens units 5007 may be provided to correspond to the imaging elements.

Further, the imaging unit 5009 may not be necessarily provided in the camera head 5005. For example, the imaging unit 5009 may be provided in the lens barrel 5003 immediately after the objective lens.

The drive unit 5011 is constituted by an actuator and moves the zoom lens and the focus lens of the lens unit 5007 by a predetermined distance along an optical axis under the control of the camera head control unit 5015. Accordingly, the magnification and the focal point of a captured image which is captured by the imaging unit 5009 can be appropriately adjusted.

The communication unit 5013 is constituted by a communication device that transmits and receives a variety of information to and from the CCU 5039. The communication unit 5013 transmits an image signal acquired from the imaging unit 5009 as RAW data to the CCU 5039 via the transmission cable 5065. At this time, it is preferable that the image signal be transmitted by optical communication to display a captured image of a site of operation with low latency. This is because since an operator 5067 performs a surgery operation while observing a state of an affected area using a captured image at the time of the surgical operation, it is necessary to display a moving image of a site of operation in real time for the purpose of a more safe and reliable surgical operation. When optical communication is used, a photoelectric conversion module that converts an electrical signal into an optical signal is provided in the communication unit 5013. The image signal is converted into an optical signal by the photoelectric conversion module and then is transmitted to the CCU 5039 via the transmission cable 5065.

Further, the communication unit 5013 receives a control signal for controlling driving of the camera head 5005 from the CCU 5039. The control signal includes, for example, information regarding imaging conditions such as information for designating a frame rate of a captured image, information for designating an exposure value at the time of imaging, and/or information for designating the magnification and the focal point of a captured image. The communication unit 5013 supplies the received control signal to the camera head control unit 5015. Incidentally, the control signal from the CCU 5039 may also be transmitted by optical communication. In this case, a photoelectric conversion module that converts an optical signal into an electrical signal is provided in the communication unit 5013. The control signal is converted into an electrical signal by the photoelectric conversion module and then is supplied to the camera head control unit 5015.

Incidentally, imaging conditions such as the frame rate, the exposure value, the magnification, and the focal point are automatically set by the control unit 5063 of the CCU 5039 on the basis of the acquired image signal. That is, so-called auto exposure (AE), auto focus (AF), and auto white balance (AWB) functions are mounted in the endoscope 5001.

The camera head control unit 5015 controls driving of the camera head 5005 on the basis of a control signal received from the CCU 5039 via the communication unit 5013. For example, the camera head control unit 5015 controls driving of the imaging element of the imaging unit 5009 on the basis of information for designating the frame rate of a captured image and/or information for designating exposure at the time of imaging. Further, for example, the camera head control unit 5015 appropriately moves the zoom lens and the focus lens of the lens unit 5007 via the drive unit 5011 on the basis of information for designating the magnification and the focal point of a captured image. The camera head control unit 5015 may further have a function of storing information for identifying the lens barrel 5003 or the camera head 5005.

Incidentally, by disposing the configuration of the lens unit 5007, the imaging unit 5009, or the like in a sealed structure with high airtightness and waterproofness, the camera head 5005 can have resistance to autoclave sterilization.

The functional configuration of the CCU 5039 will be described below. The communication unit 5059 is constituted by a communication device that transmits and receives a variety of information to and from the camera head 5005. The communication unit 5059 receives an image signal transmitted from the camera head 5005 via the transmission cable 5065. At this time, the image signal can be appropriately transmitted by optical communication as described above. In this case, a photoelectric conversion module that converts an optical signal into an electrical signal is provided in the communication unit 5059 to correspond to the optical communication. The communication unit 5059 supplies an electrical signal into which the image signal has been converted to the image processing unit 5061.

Further, the communication unit 5059 transmits a control signal for controlling driving of the camera head 5005 to the camera head 5005. The control signal may also be transmitted by optical communication.

The image processing unit 5061 performs various image processing on an image signal which is RAW data transmitted from the camera head 5005. Examples of the image processing include various known signal processing such as a developing process, an image quality enhancing process (such as a band emphasizing process, super-resolution processing, a noise reduction (NR) process, and/or a camera shake correcting process), and/or an enlargement process (an electronic zoom process), and the like. Further, the image processing unit 5061 performs a detection process for an image signal for performing the AE function, the AF function, and the AWB function.

The image processing unit 5061 is constituted by a processor such as a CPU or a GPU, and the above-mentioned image processing or the detection process can be performed by allowing the processor to operate in accordance with a predetermined program. Incidentally, when the image processing unit 5061 is constituted by a plurality of GPUs, the image processing unit 5061 appropriately divides information associated with an image signal and performs image processing in parallel by the plurality of GPUs.

The control unit 5063 performs various controls for imaging a site of operation using the endoscope 5001 and display of the captured image. For example, the control unit 5063 generates a control signal for controlling driving of the camera head 5005. At this time, when imaging conditions are input by a user, the control unit 5063 generates a control signal on the basis of the input by the user. Alternatively, when the AE function, the AF function, and the AWB function are mounted in the endoscope 5001, the control unit 5063 appropriately calculates an optimal exposure value, an optimal focal distance, and a white balance on the basis of the result of the detection process in the image processing unit 5061, and generates a control signal.

Further, the control unit 5063 displays an image of a site of operation on the display device 5041 on the basis of an image signal subjected to image processing by the image processing unit 5061. At this time, the control unit 5063 recognizes various objects in the image of the site of operation using various image recognition technologies. For example, the control unit 5063 can recognize a surgical instrument such as a forceps, a specific biological part, bleeding, mist at the time of use of the energy treatment tool 5021, or the like by detecting the shape, color, or the like of an edge of an object included in the image of the site of operation. The control unit 5063 displays a variety of surgery support information to overlap the image of the site of operation using the recognition result at the time of displaying the image of the site of operation on the display device 5041. When the surgery support information is displayed to overlap and is presented to the operator 5067, it is possible to more safe and reliably perform a surgical operation.

The transmission cable 5065 that connects the camera head 5005 and the CCU 5039 to each other is an electrical signal cable corresponding to communication of an electrical signal, an optical fiber corresponding to optical communication, or a combined cable thereof.

Here, in the illustrated example, communication is performed in a wired manner using the transmission cable 5065, but communication between the camera head 5005 and the CCU 5039 maybe performed in a wireless manner. When communication therebetween is performed in a wireless manner, the transmission cable 5065 does not need to be installed in an operation room and thus it is possible to solve a problem that movement of a medical staff in the operation room is hindered by the transmission cable 5065.

An example of the endoscopic surgery system 5000 to which the technology according to the present disclosure can be applied has been described above. Incidentally, the endoscopic surgery system 5000 has been described herein as an example, but a system to which the technology according to the present disclosure can be applied is not limited to the example. For example, the technology according to the present disclosure may be applied to an examination flexible endoscope system or a microscope surgery system.

The technology according to the present disclosure can be suitably applied to the display device 5041 among the above-mentioned elements. It may be more suitable for an operator that an image which is displayed on the display device 5041 is displayed in a vertically or laterally inverted state using the flip function depending on the position at which the operator 5067 stands, the arrangement of the display device 5041, the direction of the endoscope 5001, or the like.

By applying the technology according to the present disclosure to this case, a user can simply understand that flip setting is turned on or what behavior is performed when flip setting is turned on by inverting a screen associated with setting of the flip function which is displayed in an OSD image, a menu, or the like in the predetermined direction and displaying the inverted screen when the flip function of inverting a display direction of a through-image on the display device 5041 to a predetermined direction is set to ON by an input from the input device 5047 or the like, and it is thus possible to improve usability of the endoscopic surgery system 5000.

Further, the present technology may be embodied the following configurations.

(1)
   An image processing device including:
   a setting unit that sets a flip function of inverting a display direction of an image to a predetermined direction to ON or OFF; and
   a control unit that inverts a screen associated with setting of the flip function in the predetermined direction and displays the inverted screen when an instruction to set the flip function to ON is received.
(2)
   The image processing device according to (1), in which the control unit inverts the screen associated with setting of the flip function in a vertical direction and displays the inverted screen when an instruction to set the flip function to ON is received.
(3)
   The image processing device according to (1) or (2), in which the control unit does not display the screen associated with setting of the flip function when the flip function is set to OFF.
(4)
   The image processing device according to any one of (1) to (3), in which the screen associated with setting of the flip function is an explanation screen for the flip function.
(5)
   The image processing device according to (4), in which explanation for behavior of an operation unit when the flip function is set to ON is displayed on the explanation screen.
(6)
   The image processing device according to any one of (1) to (5), in which the screen associated with setting of the flip function is an ascertainment screen for setting of the flip function.
(7)
   The image processing device according to (6), in which the setting unit cancels setting of the flip function which has been performed before the ascertainment screen is displayed when cancellation of setting of the flip function is instructed in a state in which the ascertainment screen is displayed.
(8)
   The image processing device according to (7), in which a cancellation button for cancelling setting of the flip function is displayed on the ascertainment screen.
(9)
   The image processing device according to any one of (1) to (8), in which at least one of vertical inversion of an image acquired by imaging, exchange of right and left channels of collected sound acquired by sound collection, vertically inverted display of a display screen, or exchange of functions assigned to the operation units associated with different directions is performed when the flip function is set to ON.
(10)
   An image processing method including steps of:
   setting a flip function of inverting a display direction of an image to a predetermined direction to ON or OFF; and
   inverting a screen associated with setting of the flip function in the predetermined direction and displaying the inverted screen when an instruction to set the flip function to ON is received.
(11) A program causing a computer to perform a process, the process including steps of:
   setting a flip function of inverting a display direction of an image to a predetermined direction to ON or OFF; and
   inverting a screen associated with setting of the flip function in the predetermined direction and displaying the inverted screen when an instruction to set the flip function to ON is received.

### REFERENCE SIGNS LIST

- 11: Imaging device
- 22: Sound collecting unit
- 61: Display unit
- 63: Upward button
- 64: Downward button
- 65: Leftward button
- 66: Rightward button
- 131: Imaging unit
- 133: Control unit
- 135: Communication unit
- 136: Input and output terminal
- 151: Setting unit
- 152: OSD image generating unit
- 153: Display control unit
- 154: Recording control unit

## Claims

1. An image processing device comprising:
a setting unit that sets a flip function of inverting a display direction of an image to a predetermined direction to ON or OFF; and
a control unit that inverts a screen associated with setting of the flip function to the predetermined direction and displays the inverted screen when an instruction to set the flip function to ON is received.

2. The image processing device according to claim 1, wherein the control unit inverts the screen associated with setting of the flip function in a vertical direction and displays the inverted screen when an instruction to set the flip function to ON is received.

3. The image processing device according to claim 1, wherein the control unit does not display the screen associated with setting of the flip function when the flip function is set to OFF.

4. The image processing device according to claim 1, wherein the screen associated with setting of the flip function is an explanation screen for the flip function.

5. The image processing device according to claim 4, wherein explanation for behavior of an operation unit when the flip function is set to ON is displayed on the explanation screen.

6. The image processing device according to claim 1, wherein the screen associated with setting of the flip function is an ascertainment screen for setting of the flip function.

7. The image processing device according to claim 6, wherein the setting unit cancels setting of the flip function which has been performed before the ascertainment screen is displayed when cancellation of setting of the flip function is instructed in a state in which the ascertainment screen is displayed.

8. The image processing device according to claim 7, wherein a cancellation button for cancelling setting of the flip function is displayed on the ascertainment screen.

9. The image processing device according to claim 1, wherein at least one of vertical inversion of an image acquired by imaging, exchange of right and left channels of collected sound acquired by sound collection, vertically inverted display of a display screen, or exchange of functions assigned to the operation units associated with different directions is performed when the flip function is set to ON.

10. An image processing method comprising steps of:
setting a flip function of inverting a display direction of an image to a predetermined direction to ON or OFF; and
inverting a screen associated with setting of the flip function in the predetermined direction and displaying the inverted screen when an instruction to set the flip function to ON is received.

11. A program causing a computer to perform a process, the process comprising steps of:
setting a flip function of inverting a display direction of an image to a predetermined direction to ON or OFF; and
inverting a screen associated with setting of the flip function in the predetermined direction and displaying the inverted screen when an instruction to set the flip function to ON is received.
